# EUROPEAN PATENT SPECIFICATION

(11) **EP 1 939 165 B1**
(45) Date of publication and mention of the grant of the patent: **13.06.2012**
(21) Application number: 06797559.9
(22) Date of filing: 06.09.2006
(51) Int. Cl.: C07C 43/11, C07C 43/10, C08F 16/14, C10M 105/18, C10M 107/24, C10M 171/00

(54) **POLYVINYL ETHER COMPOUND**
POLYVINYL-ETHER-VERBINDUNG
COMPOSE D'ETHER DE POLYVINYLE

(30) Priority: 17.10.2005 JP 2005302047
(43) Date of publication of application: 02.07.2008
(73) Proprietor: IDEMITSU KOSAN CO., LTD., Tokyo 100-8321 (JP)
(72) Inventor: NAGAO, Satoshi, Sodegaura-shi, Chiba 299-0293 (JP); TERADA, Izumi, Sodegaura-shi, Chiba 299-0293 (JP); SHIMIZU, Nobuaki, Sodegaura-shi Chiba 299-0293 (JP); KANEKO, Masato, Ichihara-shi Chiba 299-0107 (JP)
(74) Representative: HOFFMANN EITLE
(86) International application number: PCT/JP2006/317664
(87) International publication number: WO 2007/046196

(56) References cited:
- EP-A1- 0 644 175
- EP-A1- 1 227 108
- EP-A1- 1 234 868
- WO-A1-01/19875
- GB-A- 958 893
- GB-A- 958 893
- JP-A- 08 193 196
- US-A- 5 514 288

## Description

### Technical Field

The present invention relates to a novel polyvinyl ether compound suitable for lubricating oil for a compression refrigerator, particularly for a compression refrigerator using a natural refrigerant.

### Background Art

Up to now, refrigerators such as those having a compression-refrigerating cycle of a compressor, a condenser, an expansion valve, and an evaporator use CFC (chlorofluorocarbon) and HCFC (hydrochlorofluorocarbon) as their refrigerants. In addition, many kinds of lubricating oil have been produced and employed in combination with such refrigerants. However, concerns are that the CFC compounds, which have been conventionally used as refrigerants, may destroy the ozone layer when the CFC compounds are discharged into the atmosphere and cause environmental pollution problems. In recent years, for measures against the environmental pollution, HFCs (hydrofluorocarbons), which may be alternatives for the CFC compounds, have been developed. A variety of so-called CFC substitutes including 1,1,1,2-tetrafluoroethane (R-134a) with a little fear of environmental pollution have become commercially available. However, concerns arise that the above-mentioned HFCs also cause environmental pollution problems. Thus, use of natural refrigerants without such problems and the like have been considered.

On the other hand, studies on carbon dioxide (CO₂)_{,} ammonia, and hydrocarbon gas have been made as natural refrigerants which substantially do not contribute to destruction of the ozone layer and global warming and will be provided as refrigerants in near feature.
For example, carbon dioxide (CO₂) is harmless for the environment and excellent from the viewpoint of safety for human, as well as having advantages of, for example, (i) its pressure almost at the optimal economical level; (ii) an extremely small pressure ratio, compared with that of the conventional refrigerant; (iii) an excellent adaptability to normal oil and structural materials of a machine; (iv) being available all over the place without any difficulty; and (v) extremely cheap price without the need of recovery. In addition, carbon dioxide (CO₂) has been used as refrigerants for some of the conventional refrigerators and the applications thereof as refrigerants for car air conditioners and heat pumps for hot water have been investigated in recent years.
Typically, for example, a compression refrigerator contains at least a compressor, a condenser, an expansion mechanism (e.g., an expansion valve), and an evaporator. Such a refrigerant-circulating system has a structure in which a liquid mixture of refrigerator oil, i.e., lubricating oil for refrigerant compressors, and a refrigerant circulates in this closed system. In the compression refrigerator, although it depends on the kind of the apparatus, the inside of the compressor reaches a high temperature and the inside of the refrigerating chamber reaches a low temperature in general. Thus, both the refrigerant and the lubricating oil should circulate in the system without causing phase separation within a wide temperature range from low to high temperatures.

In general, a temperature region in which both the refrigerant and the lubricating oil are compatible and not separated includes a phase separation region at the high temperature side and a phase separation region at the low temperature side. The high temperature side is in the range of -20°C or more, preferably 0°C or more, more preferably 10°C or more. The low temperatures is in the range of 10°C or less, preferably 0°C or less, more preferably -20°C or less.
When the phase separation occurs in the refrigerator at work, it will give a significant adverse effect on the life or efficiency of the apparatus. For example, when phase separation of the refrigerant and the lubricating oil occurs at a compressor part, it leads to insufficient lubrication in a moving part and causes burn out or the like, thereby significantly shortening the life of the apparatus. On the other hand, when the phase separation occurs in the evaporator, it leads to a decrease in heat exchange efficiency due to the presence of high viscous lubricating oil. The lubricating oil for the refrigerant-circulating system is employed for lubricating the moving part of the refrigerator, so its lubrication property is also obviously important. In particular, the inside of the compressor becomes a high temperature, so it can be important for the lubricating oil to have a viscosity enough to retain an oil film to be required for lubrication. The required viscosity of lubricating oil varies depending on the kind of the compressor to be used and the use conditions thereof. In general, however, the viscosity (kinematic viscosity) of lubricating oil yet to be mixed with the refrigerant is preferably 1 to 50 mm²/s, particularly preferably 5 to 20 mm²/s at 100°C. If the viscosity is lower than the defined value, a resulting oil film is thin and tends to cause insufficient lubrication. In contrast, if the viscosity is higher than the defined value, the heat exchange efficiency may be reduced. On the other hand, like a car air-conditioner, when it is designed for use in cold regions, the viscosity of lubricating oil should not be too high at low temperatures to ensure its ability of allowing the apparatus to be initiated. Therefore, the lubricating oil requires a lower pour point and a higher viscosity index. In general, the lubricating oil is required to have a pour point of -20°C or less, preferably -30°C or less, more preferably -40°C or less and a viscosity index of at least 80 or more, preferably 100 or more, more preferably 120 or more.

Further, the refrigerator oil requires various characteristics including lubricity and hydrolytic stability, as well as refrigerant compatibility and low-temperature fluidity.
The development of novel refrigerator oil suitable for use with carbon-dioxide refrigerants has been progressed. Polyalkylene glycol (PAG) has comparatively high compatibility to the carbon-dioxide refrigerant and is also excellent in low-temperature fluidity and hydrolytic stability, so it has drawn attention as one of substrates of refrigerator oil for carbon-dioxide refrigerants (see, for example, Patent Document 1).
The conventional PAG refrigerator oil described above shows compatibility to the carbon-dioxide refrigerator in a composition with a low proportion of the carbon-dioxide refrigerant, but the range of compatibility is not always sufficient. Therefore, there is a method for preparing PAG with low viscosity to provide such refrigerator oil with sufficient refrigerant compatibility. In this case, however, it tends to fall in a vicious cycle of being insufficient in lubricity and stability.
EP1227108 discloses a process for producing polyvinyl ether compounds for use as a component as lubricating oil for refrigerators, said lubricant having improved volume specific resistance while maintaining the kinematic viscosity, said polyvinyl ether compounds being obtained by polymerisation of vinyl ether compounds in the presence of a Lewis acid.
EP0644175 discloses lubricating oils for freezers that have a high compatibility with CFC alternatives and keep a good lubricating power and have a suitable volume intrinsic resistance.
Further, the conventional polyvinyl ether oil may be insufficient in viscosity index.

Patent Document 1: JP 10-46169 A

### Disclosure of the Invention

The present invention has been made under such circumstances and intends to provide a novel polyvinyl ether compound having good compatibility and a high viscosity index under atmospheric conditions of a natural refrigerant such as carbon dioxide as a refrigerant and suitable as lubricating oil for a compression refrigerator, particularly for a compression refrigerator using a natural refrigerant.
As a result of intensive studies for developing a polyvinyl ether compound having preferable characteristics as described above, the inventors of the present invention have found that a polyvinyl ether compound having a specific structure as defined in the claims can solve the above-mentioned problems.

### Brief Description of the Drawings

[Fig. 1] A ¹H-NMR chart of Compound 1 obtained in Example 1.
[Fig. 2] A ¹H-NMR chart of Compound 2 obtained in Example 2.
[Fig. 3] A ¹H-NMR chart of Compound 3 obtained in Example 3.
[Fig. 4] A ¹H-NMR chart of Compound 4 obtained in Example 4.
[Fig. 5] A ¹H-NMR chart of Compound 5 obtained in Example 5.
[Fig. 6] A ¹H-NMR chart of Compound 6 obtained in Example 6.
[Fig. 7] A ¹H-NMR chart of Compound 7 obtained in Example 7.
[Fig. 8] A ¹H-NMR chart of Compound 8 obtained in Example 8.
[Fig. 9] A ¹H-NMR chart of Compound 9 obtained in Example 9.
[Fig. 10] A ¹H-NMR chart of Compound 10 obtained in Example 10.
[Fig. 11] A ¹H-NMR chart of Compound 11 obtained in Example 11.
[Fig. 12] A ¹H-NMR chart of Compound 12 obtained in Example 12.
[Fig. 13] A ¹H-NMR chart of Compound 13 obtained in Example 13.
[Fig. 14] A ¹H-NMR chart of Compound 14 obtained in Example 14.
[Fig. 15] A ¹H-NMR chart of Compound 15 obtained in Example 15.
[Fig. 16] A ¹H-NMR chart of Compound 16 obtained in Example 16.
[Fig. 17] A ¹H-NMR chart of Compound 17 obtained in Example 17.
[Fig. 18] A ¹H-NMR chart of Compound 18 obtained in Example 18.
[Fig. 19 ] A ¹H-NMR chart of Compound 19 obtained in Example 19.
[Fig. 20] A ¹H-NMR chart of Compound 20 obtained in Example 20.

### Best Mode for carrying out the Invention

The polyvinyl ether compound of the present invention has several aspects as defined in the claims. One is a polyvinyl ether compound I and the others are polyvinyl ether compounds II, IV and VI.
First, the polyvinyl ether compound I is a polyvinyl ether compound characterized by having an alkylene glycol or polyoxyalkylene glycol unit and a vinyl ether unit in a molecule and a molecular weight in the range of 300 to 3, 000. The polyvinyl ether compound II having a molecular weight of 300 to 3,000 when it is obtained by polymerizing vinyl ether compounds in the presence of a polymerization initiator, is characterized in that at least one of the polymerization initiator and the vinyl ether compounds contains an alkylene glycol residue or a polyoxyalkylene glycol residue.
In the present invention, examples of a compound that meets the polyvinyl ether compound I or II include polyvinyl ether compounds 1 to 5 described below.

### [Polyvinyl Ether Compound 1]

Polyvinyl ether compound 1 is an ether compound having a constitutional unit represented by the general formula (I):

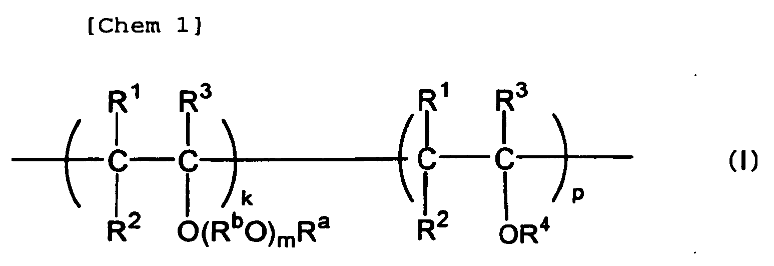

wherein R¹, R², and R³ each represent a hydrogen atom or a hydrocarbon group having 1 to 8 carbon atoms, which may be identical to or different from one another; R^{b} represents a divalent hydrocarbon group having 2 to 4 carbon atoms; R^{a} represents a hydrogen atom, an aliphatic or alicyclic hydrocarbon group having 1 to 20 carbon atoms, an aromatic group which has 1 to 20 carbon atoms and may have a substituent, an acyl group having 2 to 20 carbon atoms, or an oxygen-containing hydrocarbon group having 2 to 50 carbon atoms; R⁴ represents a hydrocarbon group having 1 to 10 carbon atoms; when there are two or more of each of R^{a}, R^{b}, and R⁴, they may be identical to or different from one another; m represents an average value of 2 to 50; k represents an average value of 1 to 50; p represents an average value of 0 to 50; when k and p each represent 2 or more, constitutional units may be in block or in random; and when there are two or more R^{b}O, they may be identical to or different from one another.
Here, specific examples of the hydrocarbon group having 1 to 8 carbon atoms represented by each of R¹, R², and R³ include: alkyl groups such as a methyl group, an ethyl group, an n-propyl group, an isopropyl group, an n-butyl group, an isobutyl group, a sec-butyl group, a tert-butyl group, various pentyl groups, various hexyl groups, various heptyl groups, and various octyl groups; cycloalkyl groups such as a cyclopentyl group, a cyclohexyl group, various methylcyclohexyl groups, various ethylcyclohexyl groups, and various dimethylcyclohexyl groups; aryl groups such as a phenyl group, various methylphenyl groups, various ethylphenyl groups, and various dimethylphenyl groups; and arylalkyl groups such as a benzyl group, various phenylethyl groups, and various methylbenzyl groups. Each of R¹, R², and R³ particularly preferably represents a hydrogen atom.

On the other hand, specific examples of the divalent hydrocarbon group having 2 to 4 carbon atoms represented by R^{b} include divalent alkylene groups such as a methylene group, an ethylene group, a propylene group, a trimethylene group, and various butylene groups.
In addition, m in the general formula (I) represents the number of repeats of R^{b}O with an average value thereof in the range of 2 to 50, preferably 2 to 20, more preferably 2 to 10, particularly preferably 2 to 5. When there are two or more R^{b}O, the two or more R^{b}O may be identical to or different from one another.
Further, k represents 1 to 50, preferably 1 to 10, more preferably 1 to 2, particularly preferably 1, while p represents 0 to 50, preferably 2 to 25, more preferably 5 to 15. When k and p each represent 2 or more, constitutional units may be in block or in random.
Examples of the aliphatic or alicyclic hydrocarbon group having 1 to 20 carbon atoms represented by R^{a} preferably include an alkyl group having 1 to 10 carbon atoms or a cycloalkyl group having 5 to 10 carbon atoms. Specific examples thereof include a methyl group, an ethyl group, an n-propyl group, an isopropyl group, an n-butyl group, an isobutyl group, a sec-butyl group, a tert-butyl group, various pentyl groups, various hexyl groups, various heptyl groups, various octyl groups, various nonyl groups, various decyl groups, a cyclopentyl group, a cyclohexyl group, various methylcyclohexyl groups, various ethylcyclohexyl groups, various propylcyclohexyl groups, and various dimethylcyclohexyl groups.

Specific examples of the aromatic group which has 1 to 20 carbon atoms and may have a substituent represented by R^{a} include: aryl groups such as a phenyl group, various tolyl groups, various ethylphenyl groups, various xylyl groups, various trimethylphenyl groups, various butylphenyl groups, and various naphthyl groups; and arylalkyl groups such as a benzyl group, various phenylethyl groups, various methylbenzyl groups, various phenylpropyl groups, and various phenylbutyl groups.
In addition, examples of the acyl group having 2 to 20 carbon atoms represented by R^{a} include an acetyl group, a propionyl group, a butyryl group, an isobutyryl group, a valeryl group, an isovaleryl group, a pivaloyl group, a benzoyl group, and toluoyl group.
Further, specific examples of the oxygen-containing hydrocarbon group having 2 to 50 carbon atoms represented by R^{a} preferably include a methoxymethyl group, a methoxyethyl group, a methoxypropyl group, a 1,1-bismethoxypropyl group, a 1,2-bismethoxypropyl group, an ethoxypropyl group, a (2-methoxyethoxy)propyl group, and a (1-methyl-2-methoxy)propyl group.
R^{a} preferably represents an alkyl group having 1 to 4 carbon atoms.

In the general formula (I), specific examples of the hydrocarbon group having 1 to 10 carbon atoms represented by R⁴ include: alkyl groups such as a methyl group, an ethyl group, an n-propyl group, an isopropyl group, an n-butyl group, an isobutyl group, various pentyl groups, various hexyl groups, various heptyl groups, various octyl groups, various nonyl groups, and various decyl groups; cycloalkyl groups such as a cyclopentyl group, a cyclohexyl group, various methylcyclohexyl groups, various ethylcyclohexyl groups, various propylcyclohexyl groups, and various dimethylcyclohexyl groups; aryl groups such as a phenyl group, various methylphenyl groups, various ethylphenyl groups, various dimethylphenyl groups, various propylphenyl groups, various trimethylphenyl groups, various butylphenyl groups, and various naphthyl groups; and arylalkyl groups such as a benzyl group, various phenylethyl groups, various methylbenzyl groups, various phenylpropyl groups, and various phenylbutyl groups.
Further, each of R¹ to R³, R^{a}, R^{b}, m, and R¹ to R⁴ may be identical to or different from one another in every constitutional unit.

The polyvinyl ether compound can be obtained using as an initiator, for example, an alkylene glycol compound or a polyoxyalkylene glycol compound represented by the general formula (VII):

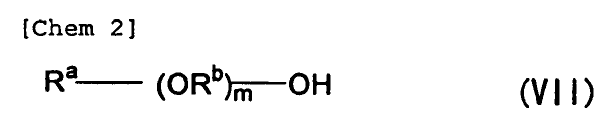

and polymerizing vinyl ether compounds represented by the general formula (VIII):

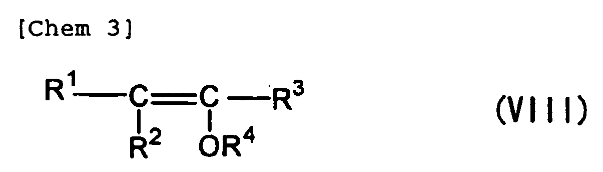

wherein R^{a}, R^{b}, and m and R¹ to R⁴ are as explained above. Specific examples of the alkylene glycol compound or the polyoxyalkylene glycol compound include: alkylene glycols such as ethylene glycol, ethylene glycol monomethyl ether, diethylene glycol, diethylene glycol monomethyl ether, triethylene glycol, triethylene glycol monomethyl ether, propylene glycol, propylene glycol monomethyl ether, dipropylene glycol, dipropylene glycol monomethyl ether, tripropylene glycol, and tripropylene glycol monomethyl ether; a polyoxyalkylene glycol; and a monoether compound thereof.

Examples of the vinyl ether compound represented by the general formula (VIII) include: vinyl ethers such as vinyl methyl ether, vinyl ethyl ether, vinyl-n-propyl ether, vinyl-isopropyl ether, vinyl-n-butyl ether, vinyl-isobutyl ether, vinyl-sec-butyl ether, vinyl-tert-butyl ether, vinyl-n-pentyl ether, and vinyl-n-hexyl ether; propenes such as 1-methoxypropene, 1-ethoxypropene, 1-n-propoxypropene, 1-isopropoxypropene, 1-n-butoxypropene, 1-isobutoxypropene, 1-sec-butoxypropene, 1-tert-butoxypropene, 2-methoxypropene, 2-ethoxypropene, 2-n-propoxypropene, 2-isopropoxypropene, 2-n-butoxypropene, 2-isobutoxypropene, 2-sec-butoxypropene, and 2-tert-butoxypropene; and butenes such as 1-methoxy-l-butene, 1-ethoxy-1-butene, 1-n-propoxy-l-butene, 1-isopropoxy-1-butene, 1-n-butoxy-l-butene, 1-isobutoxy-1-butene, 1-sec-butoxy-1-butene, 1-tert-butoxy-1-butene, 2-methoxy-1-butene, 2-ethoxy-1-butene, 2-n-propoxy-1-butene, 2-isopropoxy-1-butene, 2-n-butoxy-1-butene, 2-isobutoxy-1-butene, 2-sec-butoxy-1-butene, 2-tert-butoxy-1-butene, 2-methoxy-2-butene, 2-ethoxy-2-butene, 2-n-propoxy-2-butene, 2-isopropoxy-2-butene, 2-n-butoxy-2-butene, 2-isobutoxy-2-butene, 2-sec-butoxy-2-butene, and 2-tert-butoxy-2-butene. Those vinyl ether monomers can be produced by any known methods.

### [Polyvinyl Ether Compound 2]

Polyvinyl ether compound 2 is an ether compound having a constitutional unit represented by the general formula (II):

R^{c}-[[(OR^{d})ₐ-(A)_{b}-(OR^{f})ₑ]_{c}-R^{e}]_{d} (II)

In the general formula (II), R^{c} represents a hydrogen atom, an alkyl group having 1 to 10 carbon atoms, an acyl group having 2 to 10 carbon atoms, or a hydrocarbon group having 1 to 10 carbon atoms and having 2 to 6 binding sites; R^{d} and R^{f} represent alkylene groups having 2 to 4 carbon atoms; a and e represent average values of 0 to 50; c represents an integer of 1 to 20; R^{e} represents a hydrogen atom, an alkyl group having 1 to 10 carbon atoms, an alkoxy group having 1 to 10 carbon atoms, or an acyl group having 2 to 10 carbon atoms; and when a and/or e is 2 or more, (OR^{d}) and/or (OR^{f}) and (A) may be in random or in block.
(A) is represented by the general formula (III):

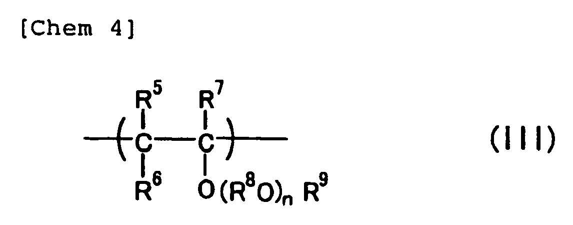

wherein R⁵, R⁶, and R⁷ each represent a hydrogen atom or a hydrocarbon group having 1 to 8 carbon atoms, which may be identical to or different from one another; R⁸ represents a divalent hydrocarbon group having 1 to 10 carbon atoms or a divalent hydrocarbon group containing ether-bonded oxygen and having 2 to 20 carbon atoms; R⁹ represents a hydrogen atom or a hydrocarbon group having 1 to 20 carbon atoms; n represents an average value of 0 to 10; when n represents 2 or more, constitutional units may be identical to or different from one another; R⁵ to R⁹ may be identical to or different from one another in every constitutional unit; and when there are two or more R⁸O, they may be identical to or different from one another. When b is 3 or more, d is an integer of 1 to 6, and both a and e are zero (0), n in one of the constitutional units A represents an integer of 1 or more.
Examples of the alkyl group having 1 to 10 carbon atoms represented by each of the above-mentioned R^{c} and R^{e} include: alkyl groups such as a methyl group, an ethyl group, an n-propyl group, an isopropyl group, an n-butyl group, an isobutyl group, various pentyl groups, various hexyl groups, various heptyl groups, various octyl groups, various nonyl groups, and various decyl groups; a cyclopentyl group; a cyclohexyl group; various methylcyclohexyl groups; various ethylcyclohexyl groups; various propylcyclohexyl groups; and various dimethylcyclohexyl groups. Examples of the acyl group having 2 to 10 carbon atoms include an acetyl group, a propionyl group, a butyryl group, an isobutyryl group, a valeryl group, an isovaleryl group, a pivaloyl group, a benzoyl group, and a toluoyl group.
Examples of the alkoxy group having 1 to 10 carbon atoms represented by R^{e} include a methoxy group, an ethoxy group, a propoxy group, abutoxygroup, a pentyloxy group, ahexyloxygroup, aheptyloxy group, an octyloxy group, a nonyloxy group, and a decyloxy group.

Examples of the hydrocarbon group having 1 to 10 carbon atoms and having 2 to 6 binding sites represented by R^{c} include residues obtained by removing hydroxy groups from polyhydric alcohols such as ethylene glycol, diethylene glycol, propylene glycol, dipropylene glycol, polypropylene glycol, neopentyl glycol, trimethylolethane, trimethylolpropane, glycerine, ditrimethylolpropane, diglycerine, pentaerythritol, dipentaerythritol, and sorbitol.
Example of the alkylene group having 2 to 4 carbon atoms represented by R^{d} include an ethylene group, a propylene group, a trimethylene group, and various butylene groups.

In the general formula (III), examples of the hydrocarbon group having 1 to 8 carbon atoms represented by each of R⁵ to R⁷ include: alkyl groups such as a methyl group, an ethyl group, an n-propyl group, an isopropyl group, an n-butyl group, an isobutyl group, various pentyl groups, various hexyl groups, various heptyl groups, and various octyl groups; cycloalkyl groups such as a cyclopentyl group, a cyclohexyl group, various methylcyclohexyl groups, various ethylcyclohexyl groups, and various dimethylcyclohexyl groups; aryl groups such as a phenyl group, various methylphenyl groups, various ethylphenyl groups, and various dimethylphenyl groups; and arylalkyl groups such as a benzyl group, various phenylethyl groups, and various methylbenzyl groups. Each of R⁵, R⁶, and R⁷ particularly preferably represents a hydrogen atom.

Specific examples of the divalent hydrocarbon group having 1 to 10 carbon atoms represented by R⁸ include: divalent aliphatic groups such as a methylene group, an ethylene group, a phenylethylene group, a 1,2-propylene group, a 2-phenyl-1,2-propylene group, a 1,3-propylene group, various butylene groups, various pentylene groups, various hexylene groups, various heptylene groups, various octylene groups, various nonylene groups, and various decylene groups; alicyclic groups each having two biding sites on alicyclic hydrocarbon, such as cyclohexane, methylcyclohexane, ethylcyclohexane, dimethylcyclohexane, and propylcyclohexane; divalent aromatic hydrocarbon groups such as various phenylene groups, various methylphenylene groups, various ethylphenylene groups, various dimethylphenylene groups, and various naphthylene groups; alkyl aromatic groups each having a monovalent biding site on each of an alkyl group portion and an aromatic group portion of an alkyl aromatic hydrocarbon such as toluene, xylene, or ethylbenzene; and alkyl aromatic groups each having a binding site on an alkyl group portion of a polyalkyl aromatic hydrocarbon such as xylene and diethylbenzene. Among them, the aliphatic groups having 2 to 4 carbon atoms are particularly preferable.

In addition, specific examples of the divalent hydrocarbon group containing ether-bonded oxygen and having 2 to 20 carbon atoms represented by R⁸ preferably include a methoxymethylene group, a methoxyethylene group, a methoxymethylethylene group, a 1,1-bismethoxymethylethylene group, a 1,2-bismethoxymethylethylene group, an ethoxymethylethylene group, a (2-methoxyethoxy)methylethylene group, and a (1-methyl-2-methoxy)methylethylene group.

Further, specific examples of the hydrocarbon group having 1 to 20 carbon atoms represented by R⁹ include: alkyl groups such as a methyl group, an ethyl group, an n-propyl group, an isopropyl group, an n-butyl group, an isobutyl group, a sec-butyl group, a tert-butyl group, various pentyl groups, various hexyl groups, various heptyl groups, various octyl groups, various nonyl groups, and various decyl groups; cycloalkyl groups such as a cyclopentyl group, a cyclohexyl group, various methylcyclohexyl groups, various ethylcyclohexyl groups, various propylcyclohexyl groups, and various dimethylcyclohexyl groups; aryl groups such as a phenyl group, various methylphenyl groups, various ethylphenyl groups, various dimethylphenyl groups, various propylphenyl groups, various trimethylphenyl groups, various butylphenyl groups, and various naphthyl groups; and arylalkyl groups such as a benzyl group, various phenylethyl groups, various methylbenzyl groups, various phenylpropyl groups, and various phenylbutyl groups.
Polyvinyl compound 2 represented by the above-mentioned general formula (II) may be preferably one in which R^{c} is a hydrogen atom, a = 0, c = 1, and d = 1 or one in which R^{e} is a hydrogen atom, e = 0, and c = 1, or one that satisfies both of them in terms of the characteristics thereof when each of them is used as lubricating oil.
Further, preferable is one in which each of R⁵ to R⁷ is a hydrogen atom, n has an average value of 0 to 4 and any one of n is one or more, R⁸ is a hydrocarbon group having 2 to 4 carbon atoms.

### [Polyvinyl Ether Compound 3]

Polyvinyl ether compound 3 is an ether compound having a structure represented by the general formula (IV):

R^{c}-[(OR^{d})ₐ-(A)_{b}-(OR^{f})ₑ]_{d}-R^{g} (IV)

In the general formula (IV), each of R^{c}, R^{d}, A, a, b, d, and e is the same as each of the general formula (II); and R^{g} represents a hydrogen atom, an alkyl group having 1 to 10 carbon atoms, an alkoxy group having 1 to 10 carbon atoms, an acyl group having 2 to 10 carbon atoms, or a hydrocarbon group having 1 to 10 carbon atoms and having 2 to 6 binding sites; when a and/or e is 2 or more, OR^{d} and/or OR^{f} and A may be in random or in block; and when each of a and e is zero (0), n represents an integer of 1 or more in one of the constitutional units A.
Examples of the alkylene group having 2 to 4 carbon atoms represented by R^{f} include an ethylene group, a propylene group, a trimethylene group, and various butylene groups.
Among R^{g}, the alkyl group having 1 to 10 carbon atoms, the acyl group having 2 to 10 carbon atoms, and the hydrocarbon groups having 1 to 10 carbon atoms and having 2 to 6 binding sites may be the same groups as those exemplified in the description about R^{c} in the general formula (II).
Further, among R^{g}, the alkoxy group having 1 to 10 carbon atoms may be the same groups as those exemplified in the description about R^{e} in the general formula (II).
Polyvinyl ether compound 3 represented by the above-mentioned general formula (IV) may be preferably one in which R^{c} is a hydrogen atom and a = 0, one in which R^{g} is a hydrogen atom, d = 1, and e = 0, or one that satisfies both of them in terms of the characteristics thereof when each of them is used as lubricating oil.
Further, preferable is one in which each of R⁵ to R⁷ is a hydrogen atom, n has an average value of 0 to 4 and any one of n is one or more, and R⁸ is a hydrocarbon group having 2 to 4 carbon atoms.

### [Polyvinyl Ether Compound 4]

Polyvinyl ether compound 4 is an ether compound having a structure represented by the general formula (VI):

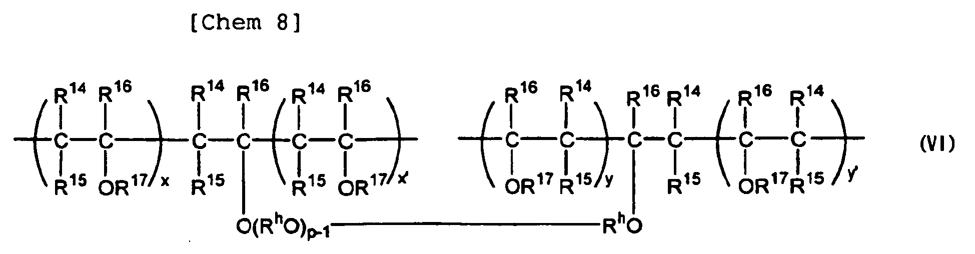

wherein R¹⁴, R¹⁵, and R¹⁶ each represent a hydrogen atom or a hydrocarbon group having 1 to 8 carbon atoms, which may be identical to or different from one another; R^{h} represents a divalent hydrocarbon group having 2 to 4 carbon atoms; R¹⁷ represents a hydrocarbon group having 1 to 10 carbon atoms; when there are two or more of each of R^{h} and R¹⁷, they may be identical to or different from one another; p represents 1 to 50; x+x' and y+y' each represent 1 to 50; and each of x, x', y, and y' represents 2 or more, constitutional units may be in block or in random.
Among R¹⁴, R¹⁵, and R¹⁶, the hydrocarbon group having 1 to 8 carbon atoms may be the same group as one exemplified in the description about R¹ to R³ in the above-mentioned general formula (I). The hydrocarbon group having 1 to 10 carbon atoms represented by R¹⁷ may be the same group as one exemplified in the description about R⁴ in the above-mentioned general formula (I). In addition, specific examples of the divalent hydrocarbon group having 2 to 4 carbon atoms represented by R^{h} include divalent alkylene groups such as a methylene group, an ethylene group, a propylene group, a trimethylene group, and various butylene groups.

The polyvinyl ether compound 4 can be obtained by using as an initiator alkylene glycol or polyoxyalkylene glycol represented by the general formula (XVI):

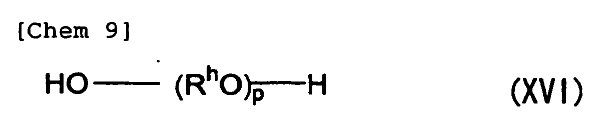

wherein R^{h} and p are identical with those described above, and polymerizing vinyl ether compounds represented by the general formula (XVII) :

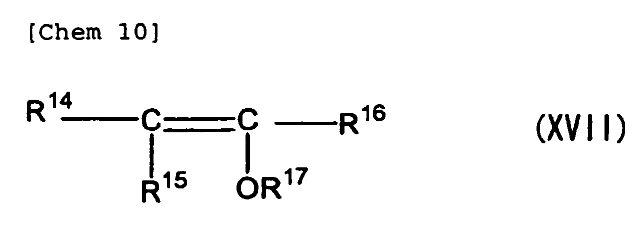

wherein R¹⁴ to R¹⁷ are identical with those described above.

Examples of the alkylene glycol or polyoxyalkylene glycol represented by the above-mentioned general formula (XVI) include ethylene glycol, diethylene glycol, triethylene glycol, polyethylene glycol, propylene glycol, dipropylene glycol, polypropylene glycol, tetramethylene glycol, and neopentyl glycol.
On the other hand, the vinyl ether compound represented by the general formula (XVII) maybe any of the same compounds exemplified in the description about the vinyl ether compound represented by the above-mentioned general formula (VIII).

The polyvinyl ether compound can be generally produced by radical polymerization, cationic polymerization, radiation polymerization, or the like of the corresponding vinyl ether compounds and optionally hydrocarbon monomers each having an olefinic double bond. For example, a polymerization product of the vinyl ether monomers can be obtained through polymerization by a method described below. For initiating the polymerization, any of combinations of Broensted acids, Lewis acids, or organic metal compounds with adducts of carboxylic acid with water, alcohols, phenols, acetals, or vinyl ethers can be used. Examples of the Broensted acids include hydrofluoric acid, hydrochloric acid, hydrobromic acid, hydroiodic acid, nitric acid, sulfuric acid, trichloroacetic acid, and trifluoroacetic acid. Examples of the Lewis acids include boron trifluoride, aluminum trichloride, aluminum tribromide, tin tetrachloride, zinc dichloride, and ferric chloride. Among those Lewis acids, boron trifluoride is particularly preferable. In addition, examples of the organic metal compounds include diethyl aluminum chloride, ethyl aluminum chloride, and diethyl zinc.

The adducts of water, alcohols, phenols, acetals, or vinyl ethers with carboxylic acid to be combined with the compounds can be optionally selected. Here, examples of the alcohols include: saturated aliphatic alcohols having 1 to 20 carbon atoms, such as methanol, ethanol, propanol, isopropanol, butanol, isobutanol, sec-butanol, tert-butanol, various pentanols, various hexanols, various heptanols, and various octanols; unsaturated aliphatic alcohols having 3 to 10 carbon atoms such as allyl alcohol; and alkylene glycols such as ethylene glycol, ethylene glycol monomethyl ether, diethylene glycol, diethylene glycol monomethyl ether, triethylene glycol, triethylene glycol monomethyl ether, propylene glycol, propylene glycolmonomethyl ether, dipropylene glycol, dipropylene glycol monomethyl ether, tripropylene glycol, and tripropylene glycol monomethyl ether, and mono ethers thereof. Examples of the carboxylic acids when adducts thereof with vinyl ethers are used include acetic acid, propionic acid, n-butyric acid, isobutyric acid, n-valeric acid, isovaleric acid, 2-methylbutyric acid, pivalic acid, n-caproic acid, 2,2-dimethyl butyric acid, 2-methyl valeric acid, 3-methyl valeric acid, 4-methyl valeric acid, enanthic acid, 2-methyl caproic acid, caprylic acid, 2-ethyl caproic acid, 2-n-propyl valeric acid, n-nonanoic acid, 3,5,5-trimethyl caproic acid, caprylic acid, and undecanoic acid.

The vinyl ethers when adducts thereof with carboxylic acids are used may be identical with those used in polymerization or may be different. The adducts of the vinyl ethers with the carboxylic acid can be obtained by mixing and reacting them at a temperature of about 0 to 100°C, and they can be separated by distillation or the like and then used for a reaction. Alternatively, it may be directly used for a reaction without separation.
When water, alcohol, or phenol is used, a hydrogen atom binds to the end of the polymer for polymerization initiation. In contrast, when acetal is used, a hydrogen atom or one of alkoxy groups of the acetal used can be detached. In addition, when an adduct of vinyl ether with carboxylic acid is used, an alkyl carbonyloxy group originated from a carboxylic acid portion is detached from the adduct of the vinyl ether with the carboxylic acid.

On the other hand, when any of water, alcohols, phenols, and acetals is used, the end of the polymer for terminating the polymerization becomes acetal, olefin, or aldehyde. In addition, in the case of an adduct of vinyl ether with carboxylic acid, it becomes carboxylic acid ester of hemiacetal. The ends of the polymer thus obtained can be converted into desired groups by a method known in the art. Examples of the desired groups include residues such as saturated hydrocarbon, ether, alcohol, ketone, nitrile, and amide. Among them, the residues such as saturated hydrocarbon, ether, and alcohol are preferable.
In the present invention, the polyvinyl ether compound 1 can be obtained using as an initiator an acetal compound represented by the general formula (IX):

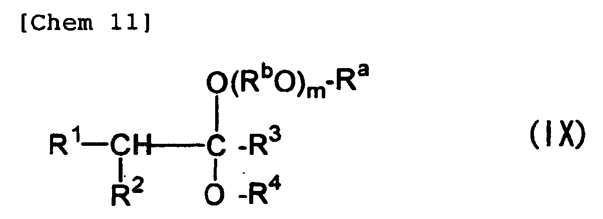

and polymerizing vinyl ether compounds of the above-mentioned general formula (VIII). In the formula (IX), R¹ to R⁴, R^{a}, R^{b}, and m are identical with those described above.
Specific examples of the acetal compound represented by the general formula (IX) include acetaldehyde dimethyl acetal, acetaldehyde diethyl acetal, acetaldehyde methylethyl acetal, acetaldehyde di-n-propyl acetal, acetaldehyde methyl-n-propyl acetal, acetaldehyde ethyl-n-propyl acetal, acetaldehyde diisopropyl acetal, acetaldehyde methyl isopropyl acetal, acetaldehyde ethyl isopropyl acetal, acetaldehyde-n-propyl isopropyl acetal, acetaldehyde di-n-butyl acetal, acetaldehyde methyl-n-butyl acetal, acetaldehyde ethyl-n-butyl acetal, acetaldehyde-n-propyl-n-butyl acetal, acetaldehyde isopropyl-n-butyl acetal, acetaldehyde diisobutyl acetal, acetaldehyde methyl isobutyl acetal, acetaldehyde ethyl isobutyl acetal, acetaldehyde-n-propyl isobutyl acetal, acetaldehyde isopropyl isobutyl acetal, acetaldehyde-n-butyl isobutyl acetal, acetaldehyde di-sec-butyl acetal, acetaldehyde methyl-sec-butyl acetal, acetaldehyde ethyl-sec-butyl acetal, acetaldehyde-n-propyl-sec-butyl acetal, acetaldehyde isopropyl-sec-butylacetal, acetaldehyde-n-butyl-sec-butyl acetal, acetaldehyde isobutyl-sec-butyl acetal, acetaldehyde di-tert-butyl acetal, acetaldehyde methyl-tert-butyl acetal, acetaldehyde ethyl-tert-butyl acetal, acetaldehyde-n-propyl-tert-butyl acetal, acetaldehyde isopropyl-tert-butyl acetal, acetaldehyde-n-butyl-tert-butyl acetal, acetaldehyde isobutyl-tert-butyl acetal, acetaldehyde-sec-butyl-tert-butyl acetal, acetals of various propionaldehydes, and acetals of various butylaldehydes.

Further, the polyvinyl ether compound 1 can be obtained such that an acetal compound represented by the above-mentioned general formula (IX) and obtained by reacting one molecule of an alkylene glycol or polyoxyalkylene glycol compound represented by the general formula (VII) with one molecule of a vinyl ether compound represented by the above-mentioned general formula (VIII) is isolated and used or directly used as an initiator, followed by polymerizing the vinyl ether compounds represented by the general formula (VIII).
In addition, the polyvinyl ether compound 1 can be prepared using as an initiator an acetal compound represented by the general formula (X):

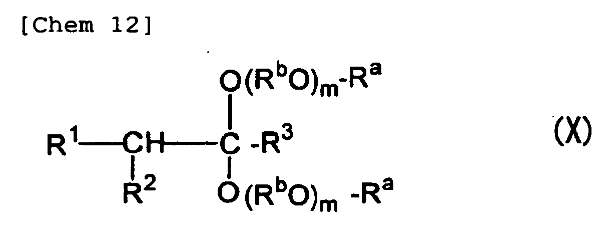

and polymerizing vinyl ether compounds represented by the general formula (VIII). In the general formula (X), R¹ to R³, R^{a}, R^{b}, and m are identical with those described above.
Examples of the acetal compound of this kind include acetaldehyde di(2-methoxyethyl) acetal, acetaldehyde di(2-methoxy-lmethylethyl) acetal, acetaldehyde di[2-(2-methoxyethoxy)ethyl] acetal, and acetaldehyde [2-(2-methoxyethoxy)-1-methylethyl] acetal.
Further, the polyvinyl ether compound 1 can be obtained such that an acetal compound represented by the above-mentioned general formula (X) and obtained by reacting one molecule of an alkylene glycol or polyoxyalkylene glycol compound represented by the general formula (VII) with one molecule of a vinyl ether compound represented by the general formula (XI):

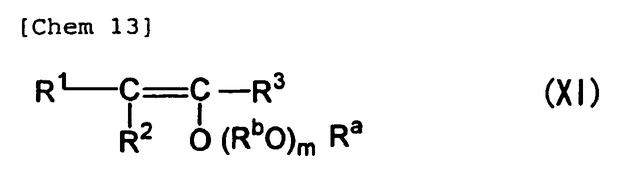

is isolated and used or directly used as an initiator, followed by polymerizing the vinyl ether compounds represented by the general formula (VIII).
In the general formula (XI), R¹ to R³, R^{a}, R^{b}, and m are identical with those described above.
Thepolyvinyl ether compound 1 may be a polyvinyl ether compound having one end represented by the general formula (XII) or (XIII) :

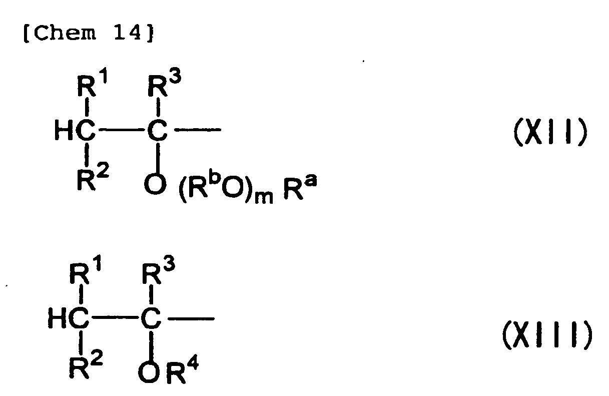

wherein R¹ to R⁴, R^{a}, R^{b}, and m are identical with those described above, and the other end represented by the general formula (XIV) or (XV) :

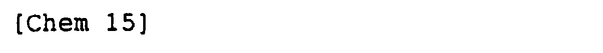

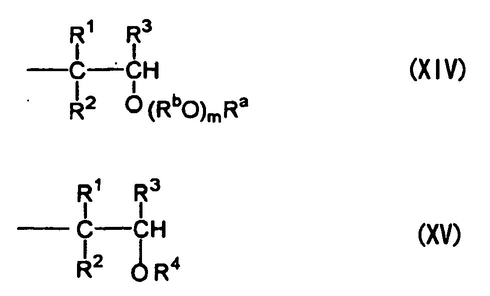

wherein R¹ to R⁴, R^{a}, R^{b}, and m are identical with those described above.

Among those polyvinyl ether compounds 1, those exemplified below are suitable as lubricating oil for a compression refrigerator.
(1) A compound having a structure with one end represented by the general formula (XII) or (XIII) and the other end represented by the general formula (XIV) or (XV), where all of R¹, R², and R³ in the general formula (I) are hydrogen atoms, the average value of m is 1 to 20, R^{b} is a divalent hydrocarbon group having 2 to 4 carbon atoms, R^{a} is an alkyl group having 1 to 10 carbon atoms, and R⁴ is a hydrocarbon group having 1 to 10 carbon atoms.
(2) A compound having a structure with one end represented by the general formula (XII) and the other end represented by the general formula (XV), where all of R¹, R², and R³ in the general formula (I) are hydrogen atoms, the average value of m is 1 to 20, R^{b} is a divalent hydrocarbon group having 2 to 4 carbon atoms, R^{a} is an alkyl group having 1 to 10 carbon atoms, and R⁴ is a hydrocarbon group having 1 to 10 carbon atoms.
(3) A compound having a structure with one end represented by the general formula (XIII) and the other end represented by the general formula (XIV) , where all of R¹, R², and R³ in the general formula (I) are hydrogen atoms, the average value of m is 1 to 20, R^{b} is a divalent hydrocarbon group having 2 to 4 carbon atoms, R³ is an alkyl group having 1 to 10 carbon atoms, and R⁴ is a hydrocarbon group having 1 to 10 carbon atoms.

The polymerization of vinyl ether monomers represented by the above-mentioned general formula (VIII) or (XVII) can be initiated at a temperature in a range from -80 to 150°C depending on the kinds of raw materials and initiators. Typically, it can be carried out at a temperature in a range from -80 to 50°C. In addition, a polymerization reaction can be completed within about 10 seconds to 10 hours after initiating the reaction. The molecular weight of the polymer can be adjusted by increasing the amount of an alcohol or an acetal compound with respect to the vinyl ether monomer, to thereby obtain a polymer having a low average molecular weight.
Further, a polymer having a low average molecular weight can be obtained by increasing the amount of Broensted acid or Lewis acid. This polymerization reaction is usually performed in the presence of a solvent. The solvent may be any of solvents that dissolve the amounts of reaction raw materials required and are inert to the reaction. Examples thereof which can be preferably used include, but not particularly limited to: hydrocarbon solvents such as hexane, benzene, and toluene; and ether solvents such as ethyl ether, 1,2-dimethoxyethane, and tetrahydrofuran. Further, this polymerization reaction can be terminated by the addition of alkali. After completion of the polymerization reaction, if required, common separation and purification procedures may be carried out to obtain a polyvinyl ether compound of interest.
The polyvinyl ether compound of the present invention has good compatibility and a high viscosity index under atmospheric conditions of a natural refrigerant such as carbon dioxide as a refrigerant, so that it is suitable as lubricating oil for compression refrigerator or lubricating oil for water heater.

### EXAMPLES

Next, the present invention will be described in more detail with reference to examples. However, the present invention is not limited by the examples described below.

### (Catalyst Preparation Example 1)

A 2-liter autoclave made of SUS316L was fed with 6 g of a nickel diatomaceous earth catalyst (a product of Nikki Chemical Co., Ltd. ; N113) and 300 g of isooctane. The autoclave was purged with nitrogen and then purged with hydrogen, followed by increasing the temperature therein while the pressure of hydrogen was adjusted to 3.0 MPaG. After retaining the autoclave at 140°C for 30 minutes, the autoclave was cooled to room temperature. The autoclave was purged with nitrogen and then fed with 10 g of acetaldehyde diethyl acetal. The autoclave was purged with nitrogen again and then purged with hydrogen, followed by increasing the temperature therein while the pressure of hydrogen was adjusted to 3.0 MPaG. After retaining the autoclave at 130°C for 30 minutes, the autoclave was cooled to room temperature. A decrease in hydrogen pressure was confirmed as the reaction of acetaldehyde diethyl acetal proceeded while an increase in temperature allowed an increase in inner pressure of the autoclave. When the pressure decreased to 3.0 MPaG or less, hydrogen was additionally supplied, thereby keeping the reaction pressure at 3.0 MPaG. The autoclave was cooled to room temperature and then depressurized. Subsequently, the autoclave was purged with nitrogen and then depressurized.

### Example 1

### (Production of Vinyl Ether Polymerization Product)

A 1-liter separable flask made of glass was fed with 60.5 g of isooctane, 37.1g (2.50×10⁻¹mol) of dipropylene glycol monomethyl ether, and 0.296 g of a boron trifluoride diethyl ether complex. Subsequently, 216.3 g (3.00 mol) of ethyl vinyl ether was added over 3 hours and 10 minutes. A reaction was exothermic, so a reaction solution was kept at 25°C by placing the flask in an ice-water bath. After that, the reaction solution was transferred to a 1-liter separation funnel and washed with 50 ml of a 5% by mass aqueous solution of sodium hydroxide and then washed with 100 ml of distilled water six times, followed by removing the solvent and light components using a rotary evaporator under reduced pressure. Consequently, 246.3 g of a crude product was obtained.
The crude product had kinematic viscosities of 114.9 mm²/s at 40°C and 11.45 mm²/s at 100°C.

### (Hydrogenation of Vinyl Ether Polymerization Product)

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, followed by increasing the temperature therein while the pressure of hydrogen was adjusted to 3.0 MPaG. After retaining the autoclave at 160°C for 3 hours, the autoclave was cooled to room temperature. A decrease in hydrogen pressure was confirmed as the reaction proceeded while an increase in temperature allowed an increase in inner pressure of the autoclave. When the pressure of hydrogen decreases, hydrogen was suitably supplied, thereby keeping the inside of the autoclave at 3.0 MPaG. The autoclave was purged with nitrogen and then depressurized, followed by recovering a reaction solution and then removing the catalyst therefrom by filtration.

A filtrate was subjected to a rotary evaporator under reduced pressure to remove the solvent and light components . Consequently, Compound 1 was obtained. The yield thereof was 89.1g. A theoretical structure of Compound 1 estimated from the feed is (A) R^{y} = CH (CH₃)CH₂, m = 2, R^{z} = CH₃, (B) R^{x} = CH₂CH₃, (A) / (B) molar ratio (k/p) = 1/11, k+p = 12 (average value), and a calculated molecular weight of 957, from the formula (XX) represented below.

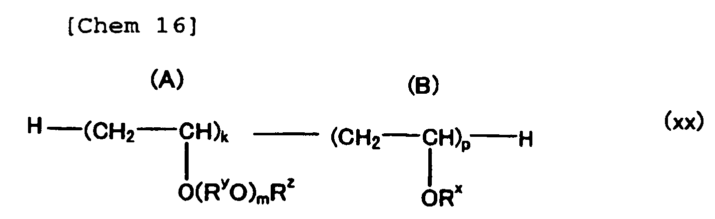

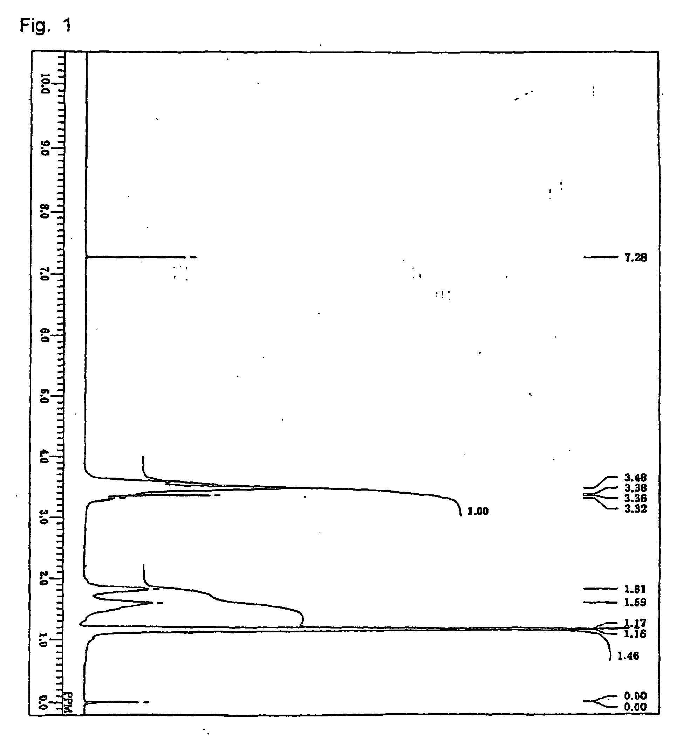

A ¹H-NMR chart of Compound 1 obtained by the NMR analysis with a measurement process described later is as shown in Fig. 1.

### Example 2

### (Production of Vinyl Ether Polymerization Product)

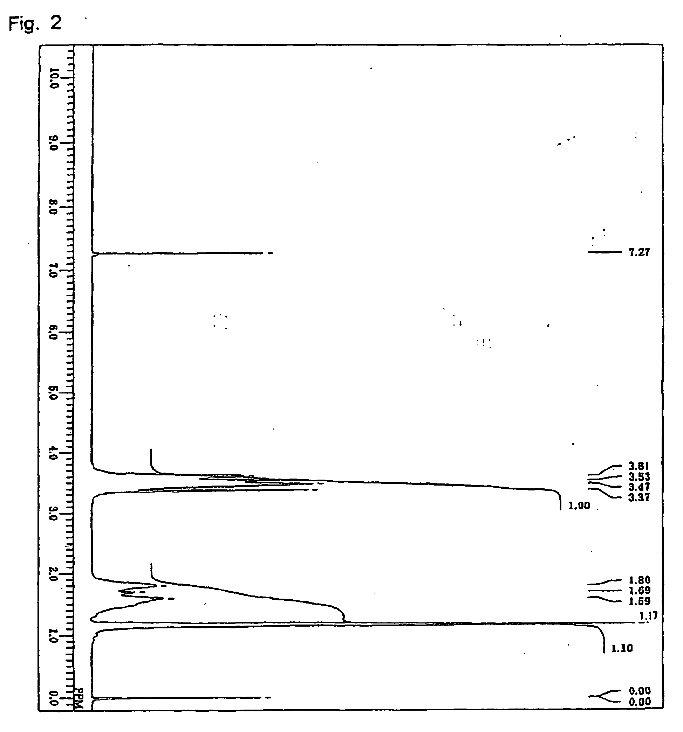

A 1-liter separable flask made of glass was fed with 60.5 g of toluene, 20.0 g (1.66×10⁻¹ mol) of diethylene glycol monomethyl ether, and 0.196 g of a boron trifluoride diethyl ether complex. Subsequently, 24.3 g (1.66×10⁻¹ mol) of diethylene glycol methyl vinyl ether was added over 20 minutes. After continuously stirring for 5 minutes without performing any other operation, 131.7 g (1.83 mol) of ethyl vinyl ether was further added over 2 hours.
After that, 171.0 g of a crude product was obtained by the same way as that of Example 1. The crude product had kinematic viscosities of 60.98 mm²/s at 40°C and 8.359 mm²/s at 100°C. (Hydrogenation of Vinyl Ether Polymerization Product)
Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 2 was obtained by the same way as that of Example 1. The yield thereof was 90.7 g. A theoretical structure of Compound 2 estimated from the feed is (A) R^{y} = CH₂CH₂, m = 2, R^{z} = CH₃, (B) R^{x} = CH₂CH₃, (A)/(B) molar ratio (k/p) = 2/10, k+p = 12 (average value) , and a calculated molecular weight of 1,015, from the formula (XX).
A ¹H-NMR chart of Compound 2 is as shown in Fig. 2.

### Example 3

### (Production of Vinyl Ether Polymerization Product)

A 1-liter separable flask made of glass was fed with 60.5 g of isooctane, 102.0 g (3.00×10⁻¹ mol) of polypropylene glycol monobutyl ether (having an average molecular weight of about 340), and 0.355 g of a boron trifluoride diethyl ether complex. Subsequently, 173.1 g (2.40 mol) of ethyl vinyl ether was added over 2 hours and 45 minutes. After that, 260.6 g of a crude product was obtained by the same way as that of Example 1. The crude product had kinematic viscosities of 71.08 mm²/s at 40°C and 10.16 mm²/s at 100°C.

### (Hydrogenation of Vinyl Ether Polymerization Product)

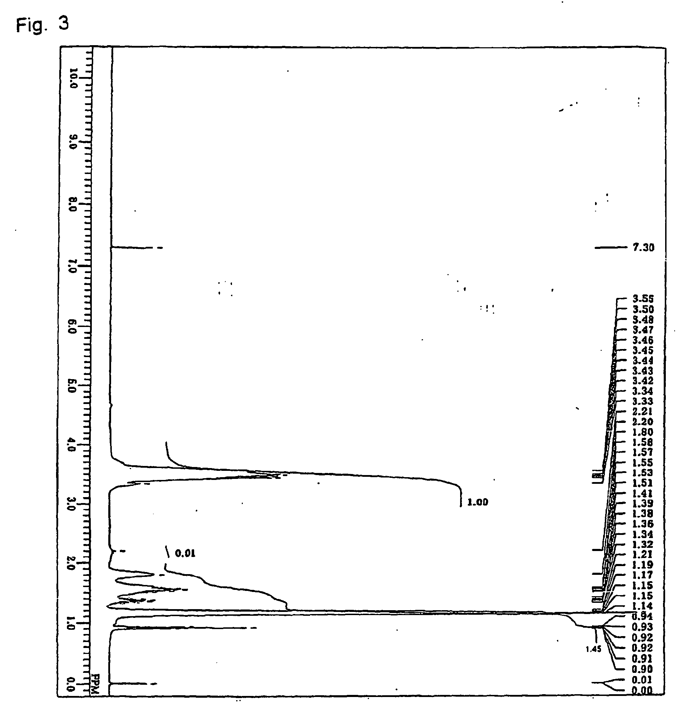

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 3 was obtained by the same way as that of Example 1. The yield thereof was 90.8 g. A theoretical structure of Compound 3 estimated from the feed is (A) R^{y} = CH(CH₃)CH₂, m = 4.6, R^{z} = CH₂CH₂CH₂CH₃. (B) R^{x} = CH₂CH₃, (A)/(B) molar ratio (k/p) = 1/7, k+p = 8 (average value), and a calculated molecular weight of 873, from the formula (XX).
A ¹H-NMR chart of Compound 3 is as shown in Fig. 3.

### Example 4

### (Production of Vinyl Ether Polymerization Product)

A 1-liter separable flask made of glass was fed with 60.5 g of isooctane, 30.0 g (2.50×10⁻¹ mol) of diethylene glycol monomethyl ether, and 0.296 g of a boron trifluoride diethyl ether complex. Subsequently, 252.5 g (3.50 mol) of ethyl vinyl ether was added over 3 hours and 39 minutes. After that, 235.1 g of a crude product was obtained by the same way as that of Example 1. The crude product had kinematic viscosities of 120.0 mm²/s at 40°C and 12.05 mm²/s at 100°C.

### (Hydrogenation of Vinyl Ether Polymerization Product)

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and purged with hydrogen, and then Compound 4 was obtained by the same way as that of Example 1. The yield thereof was 92.3 g. A theoretical structure of Compound 4 estimated from the feed is (A) R^{y} = CH₂CH₂, m = 2, R^{z} = CH₃, (B) R^{x} = CH₂CH₃, (A)/(B) molar ratio (k/p) = 1/13, k+p = 14 (average value), and a calculated molecular weight of 1,086, from the formula (XX).

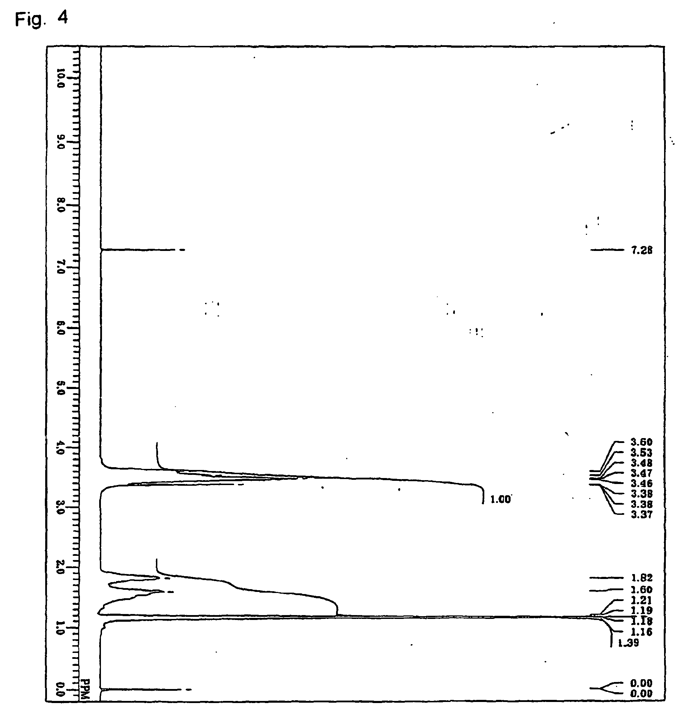

A ¹H-NMR chart of Compound 4 is as shown in Fig. 4.

### Example 5

### (Production of Vinyl Ether Polymerization Product)

A 1-liter separable flask made of glass was fed with 60.5 g of isooctane, 30.0 g (2.50×10⁻¹ mol) of diethylene glycol monomethyl ether, and 0.296 g of a boron trifluoride diethyl ether complex. Subsequently, 234.1 g (3.25 mol) of ethyl vinyl ether was added over 3 hours and 20 minutes. After that, 237. 9 g of the crude product was obtained by the same way as that of Example 1. The crude product had kinematic viscosities of 105.9 mm²/s at 40°C and 11.04 mm²/s at 100°C.

### (Hydrogenation of Vinyl Ether Polymerization Product)

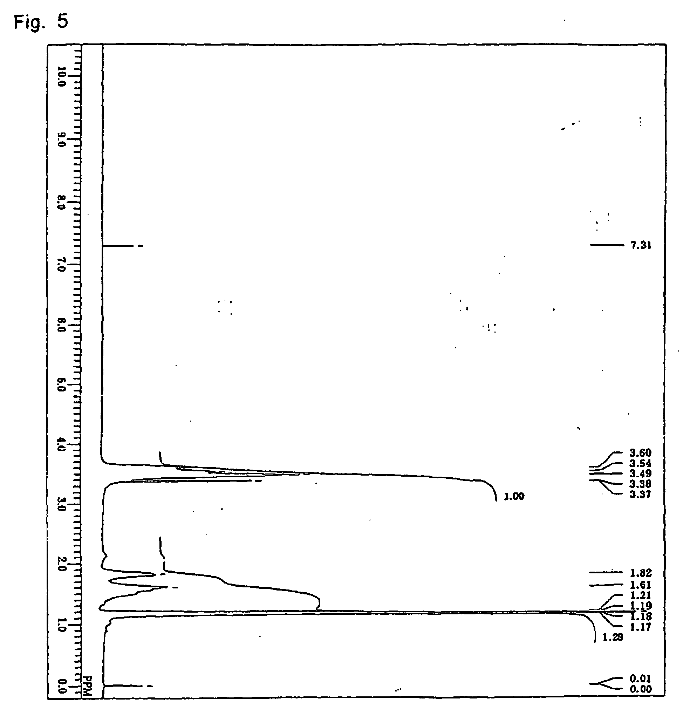

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 5 was obtained by the same way as that of Example 1. The yield thereof was 89.3 g. A theoretical structure of Compound 5 estimated from the feed is (A) R^{y} = CH₂CH₂, m = 2, R^{z} = CH₃, (B) R^{x} = CH₂CH₃, (A)/(B) molar ratio (k/p) = 1/12, k+p = 13 (average value), and a calculated molecular weight of 1,013, from the formula (XX).
A ¹H-NMR chart of Compound 5 is as shown in Fig. 5.

### Example 6

### (Production of Vinyl Ether Polymerization Product)

A 1-liter separable flask made of glass was fed with 60.5 g of toluene, 25.0 g (1.52×10⁻¹ mol) of triethylene glycol monomethyl ether, and 0.180 g of a boron trifluoride diethyl ether complex. Subsequently, 131.7 g (1.83 mol) of ethyl vinyl ether was added over 1 hour and 55 minutes. After that, 149.5 g of the crude product was obtained by the same way as that of Example 1. The crude product had kinematic viscosities of 54.91 mm²/s at 40°C and 7.617 mm²/s at 100°C.

### (Hydrogenation of Vinyl Ether Polymerization Product)

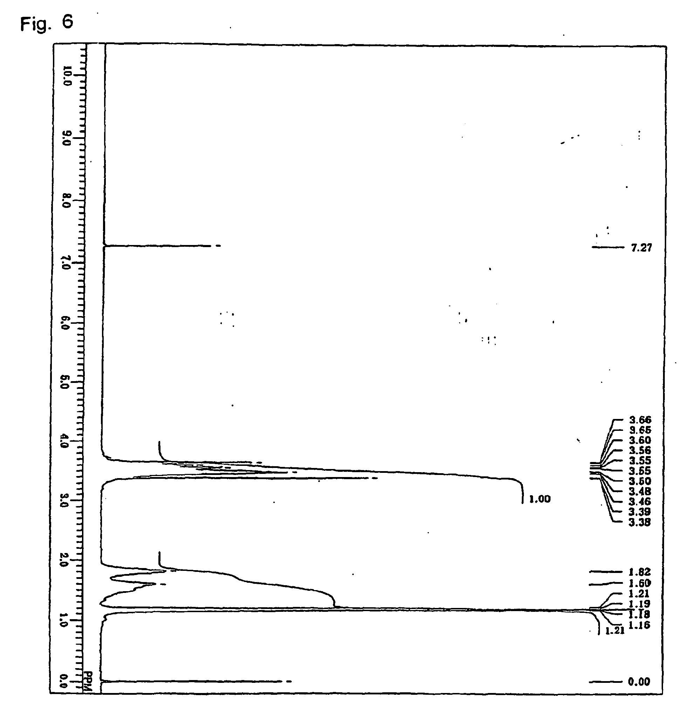

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 6 was obtained by the same way as that of Example 1. The yield thereof was 92.4 g. A theoretical structure of Compound 6 estimated from the feed is (A) R^{y} = CH₂CH₂, m = 3, R^{z} = CH₃, (B) R^{x} = CH₂CH₃, (A)/(B) molar ratio (k/p) = 1/11, k+p = 12 (average value), and a calculated molecular weight of 984, from the formula (XX).
A ¹H-NMR chart of Compound 6 is as shown in Fig. 6.

### Example 7

### (Production of Vinyl Ether Polymerization Product)

A 5-liter separable flask made of glass was fed with 520 g of isooctane, 413.0 g (2.00 mol) of tripropylene glycol monomethyl ether, and 2.37 g of a boron trifluoride diethyl ether complex. Subsequently, 1,947 g (27.0 mol) of ethyl vinyl ether was added over 4 hours and 20 minutes. After that, 2, 313 g of a crude product was obtained by the same way as that of Example 1. The crude product had kinematic viscosities of 134.9 mm²/s at 40°C and 13.15 mm²/s at 100°C.

### (Hydrogenation of Vinyl Ether Polymerization Product)

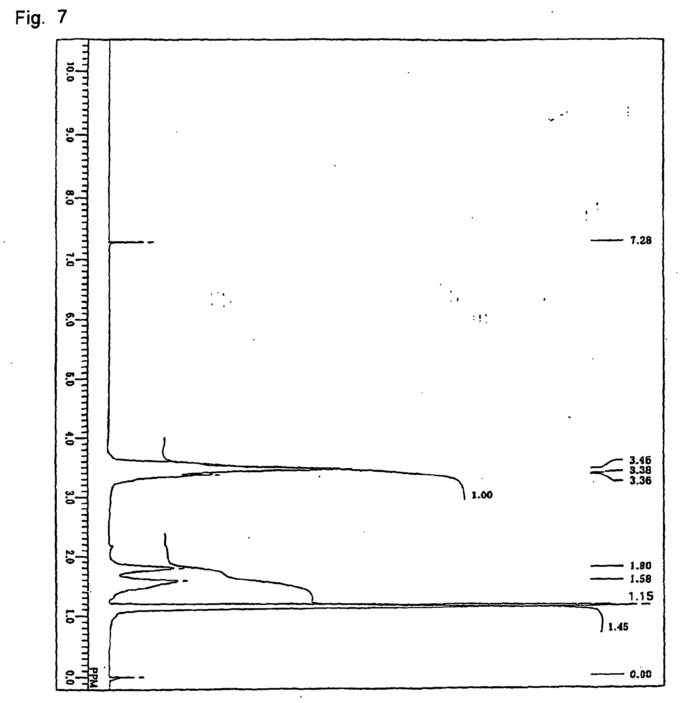

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 7 was obtained by the same way as that of Example 1. The yield thereof was 89.2 g. A theoretical structure of Compound 7 estimated from the feed is (A) R^{y} = CH(CH₃)CH₂, m = 3, R^{z} = CH₃, (B) R^{x} = CH₂CH₃, (A)/(B) molar ratio (k/p) = 1/12.5, k+p = 13.5 (average value), and a calculated molecular weight of 1,134, from the formula (XX).
A ¹H-NMR chart of Compound 7 is as shown in Fig. 7.

### Example 8

### (Production of Vinyl Ether Polymerization Product)

A 1-liter separable flask made of glass was fed with 60.5 g of isooctane, 60.0 g (1.88×10⁻¹ mol) of polypropylene glycol monomethyl ether (having an average molecular weight of about 320), and 0.222 g of a boron trifluoride diethyl ether complex. Subsequently, 120.8 g (1.68 mol) of ethyl vinyl ether was added over 1 hour and 50 minutes. After that, 174.5 g of a crude product was obtained by the same way as that of Example 1. The crude product had kinematic viscosities of 62.93 mm²/s at 40°C and 9.920 mm²/s at 100°C.

### (Hydrogenation of Vinyl Ether Polymerization Product)

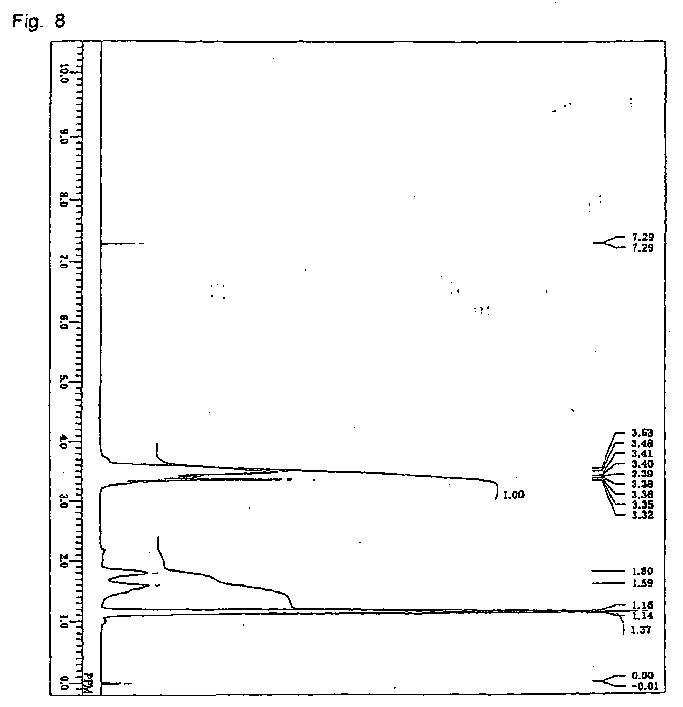

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 8 was obtained by the same way as that of Example 1. The yield thereof was 92.9 g. A theoretical structure of Compound 8 estimated from the feed is (A) R^{y} = CH(CH₃)CH₂, m = 5.0 (average value), R^{z} = CH₃, (B) R^{x} = CH₂CH₃, (A)/(B) molar ratio (k/p) = 1/8, k+p = 9 (average value), and a calculated molecular weight of 927, from the formula (XX).
A ¹H-NMR chart of Compound 8 is as shown in Fig. 8.

### Example 9

### (Production of Vinyl Ether Polymerization Product)

A 1-liter separable flask made of glass was fed with 60.5 g of isooctane, 80.0 g (1.82×10⁻¹ mol) of polypropylene glycol monomethyl ether (having an average molecular weight of about 440), and 0.218 g of a boron trifluoride diethyl ether complex. Subsequently, 92.1 g (1.28 mol) of ethyl vinyl ether was added over 1 hour and 20 minutes. After that, 164.2 g of a crude product was obtained by the same way as that of Example 1. The crude product had kinematic viscosities of 52.09 mm²/s at 40°C and 8.5.38 mm²/s at 100°C.

### (Hydrogenation of Vinyl Ether Polymerization Product)

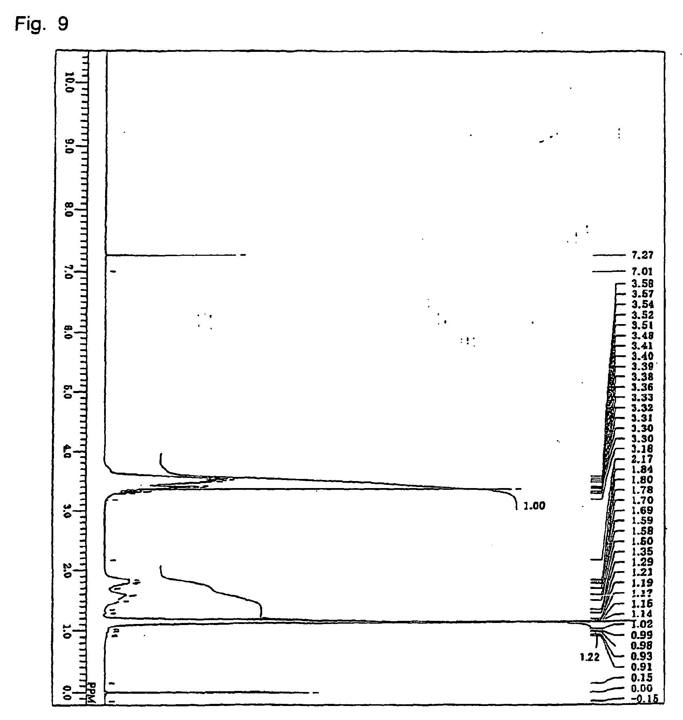

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 9 was obtained by the same way as that of Example 1. The yield thereof was 92.4 g. A theoretical structure of Compound 9 estimated from the feed is (A) R^{y} = CH(CH₃)CH₂, m = 7.0 (average value), R^{z} = CH₃, (B) R^{x} = CH₂CH₃, (A)/(B) molar ratio (k/p) = 1/6, k+p = 7 (average value), and a calculated molecular weight of 899, from the formula (XX).
A ¹H-NMR chart of Compound 9 is as shown in Fig. 9.

### Example 10

### (Production of Vinyl Ether Polymerization Product)

A 1-liter separable flask made of glass was fed with 60.5 g of isooctane, 45.0 g (1.67×10⁻¹ mol) of polypropylene glycol monomethyl ether (having an average molecular weight of about 270), and 0.202 g of a boron trifluoride diethyl ether complex. Subsequently, 135.1 g (1.87 mol) of ethyl vinyl ether was added over 2 hours. After that, 174.6 g of a crude product was obtained by the same way as that of Example 1. The crude product had kinematic viscosities of 104.5 mm²/s at 40°C and 11.81 mm²/s at 100°C.

### (Hydrogenation of Vinyl Ether Polymerization Product)

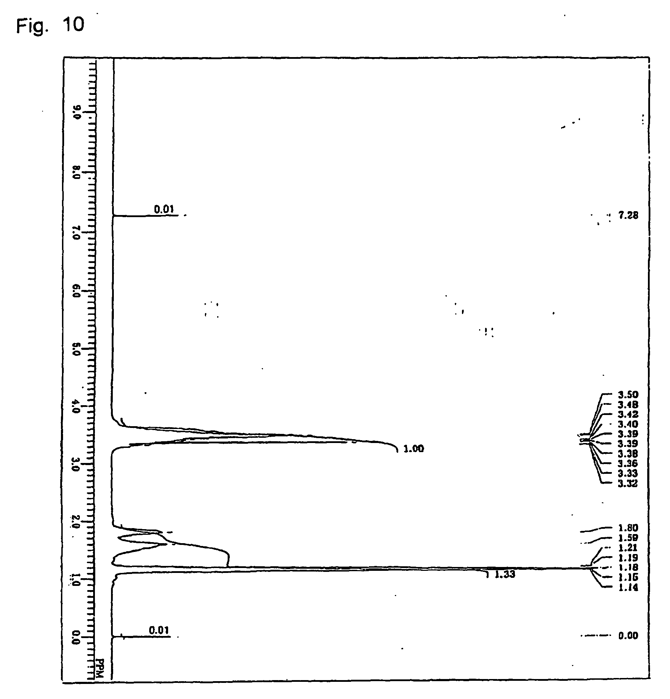

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 10 was obtained by the same way as that of Example 1. The yield thereof was 93. 7 g. A theoretical structure of Compound 10 estimated from the feed is (A) R^{y} = CH(CH₃)CH₂, m = 4.1 (average value), R^{z} = CH₃, (B) R^{x} = CH₂CH₃, (A)/(B) molar ratio (k/p) = 1/10, k+p = 11 (average value), and a calculated molecular weight of 1,018, from the formula (XX).
A ¹H-NMR chart of Compound 10 is as shown in Fig. 10.

### Example 11

### (Production of Vinyl Ether Polymerization Product)

A 1-liter separable flask made of glass was fed with 60.5 g of isooctane, 60.0 g (1.54×10⁻¹ mol) of polypropylene glycol monomethyl ether (having an average molecular weight of about 390) , and 0.187 g of a boron trifluoride diethyl ether complex. Subsequently, 113.7 g (1.58 mol) of ethyl vinyl ether was added over 1 hour and 45 minutes. After that, 168.5 g of a crude product was obtained by the same way as that of Example 1. The crude product had kinematic viscosities of 92.38 mm²/s at 40°C and 11.77 mm²/s at 100°C.

### (Hydrogenation of Vinyl Ether Polymerization Product)

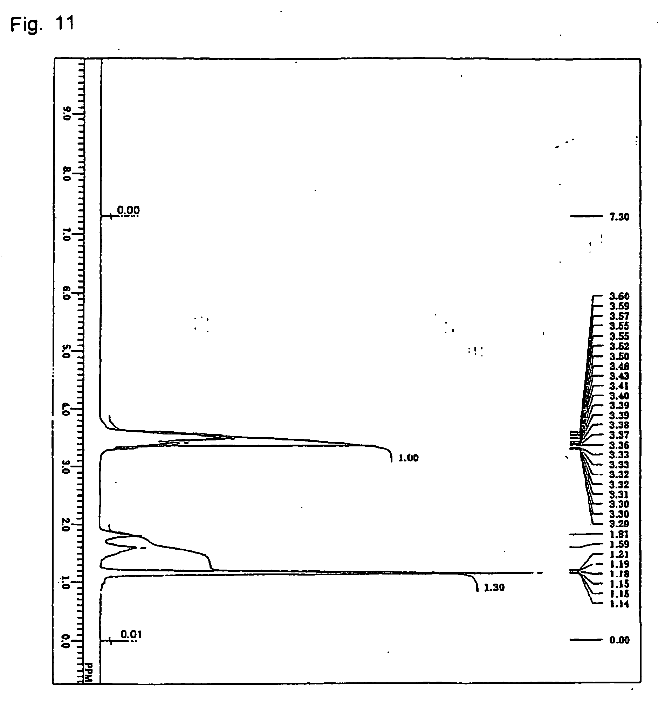

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 11 was obtained by the same way as that of Example 1. The yield thereof was 93. 5 g. A theoretical structure of Compound 11 estimated from the feed is (A) R^{y} = CH(CH₃)CH₂, m = 6.2 (average value), R^{z} = CH₃, (B) R^{x} = CH₂CH₃, (A)/(B) molar ratio (k/p) = 1/9, k+p = 10 (average value), and a calculated molecular weight of 1,068, from the formula (XX).
A ¹H-NMR chart of Compound 11 is as shown in Fig. 11.

### Example 12

### (Production of Vinyl Ether Polymerization Product)

A 1-liter separable flask made of glass was fed with 60.5 g of isooctane, 52.6 g (1.20×10⁻¹ mol) of polypropylene glycol monomethyl ether (having an average molecular weight of about 440), and 0.142 g of a boron trifluoride diethyl ether complex. Subsequently, 129.8 g (1.80 mol) of ethyl vinyl ether was added over 1 hour and 27 minutes. After that, 179.3 g of the crude product was obtained by the same way as that of Example 1. The crude product had kinematic viscosities of 227.8 mm²/s at 40°C and 21.42 mm²/s at 100°C.

### (Hydrogenation of Vinyl Ether Polymerization Product)

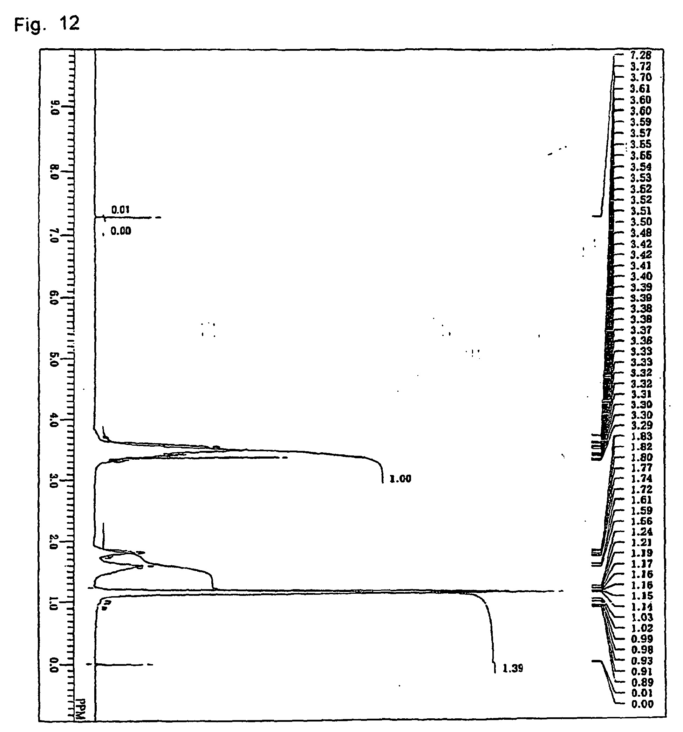

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 12 was obtained by the same way as that of Example 1. The yield thereof was 94.9 g. A theoretical structure of Compound 12 estimated from the feed is (A) R^{y} = CH(CH₃)CH₂, m = 7.0 (average value), R^{z} = CH₃, (B) R^{x} = CH₂CH₃, (A)/(B) molar ratio (k/p) = 1/14, k+p = 15 (average value), and a calculated molecular weight of 1,476, from the formula (XX).
A ¹H-NMR chart of Compound 12 is as shown in Fig. 12.

### Example 13

### (Production of Vinyl Ether Polymerization Product)

A 1-liter separable flask made of glass was fed with 60.5 g of isooctane, 73.5 g (1.15×10⁻¹ mol) of polypropylene glycol monomethyl ether (having an average molecular weight of about 640), and 0.142 g of a boron trifluoride diethyl ether complex. Subsequently, 112.5 g (1.56 mol) of ethyl vinyl ether was added over 1 hour and 15 minutes. After that, 182.3 g of a crude product was obtained by the same way as that of Example 1. The crude product had kinematic viscosities of 188.4 mm²/s at 40°C and 21.18 mm²/s at 100°C.

### (Hydrogenation of Vinyl Ether Polymerization Product)

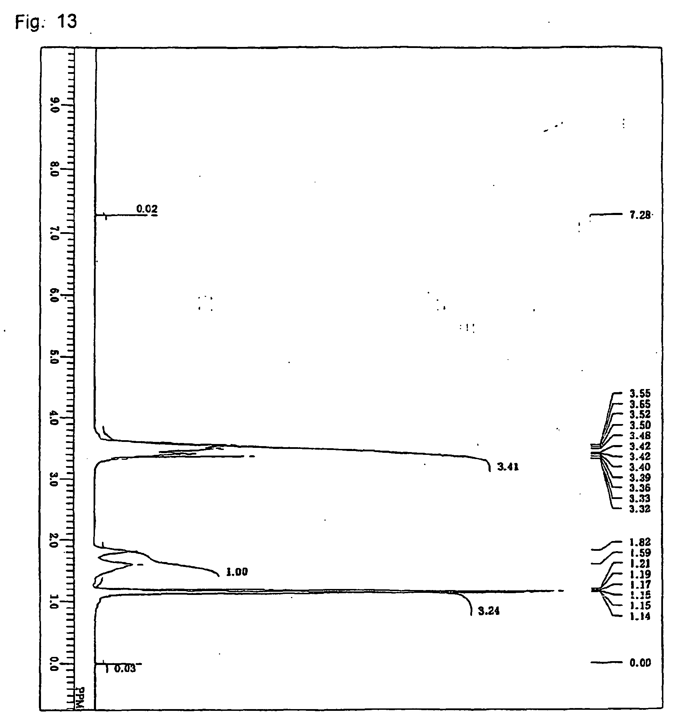

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 13 was obtained by the same way as that of Example 1. The yield thereof was 94.9g. A theoretical structure of Compound 13 estimated from the feed is (A) R^{y} = CH(CH₃)CH₂, m = 10.5 (average value), R² = CH₃, (B) R^{x} = CH₂CH₃, (A)/(B) molar ratio (k/p) = 1/12, k+p = 13 (average value), and a calculated molecular weight of 1, 533, from the formula (XX).
A ¹H-NMR chart of Compound 13 is as shown in Fig. 13.

### Example 14

### (Production of Vinyl Ether Polymerization Product)

A 1-liter separable flask made of glass was fed with 205.8 g of isooctane, 358.5 g (3.92×10⁻¹ mol) of polypropylene glycol monomethyl ether (having an average molecular weight of about 915), and 0.484 g of a boron trifluoride diethyl ether complex. Subsequently, 264.8 g (3.67 mol) of ethyl vinyl ether was added over 1 hour and 31 minutes. After that, 611.3 g of a crude product was obtained by the same way as that of Example 1. The crude product had kinematic viscosities of 137.0 mm²/s at 40°C and 19.44 mm²/s at 100°C.

### (Hydrogenation of Vinyl Ether Polymerization Product)

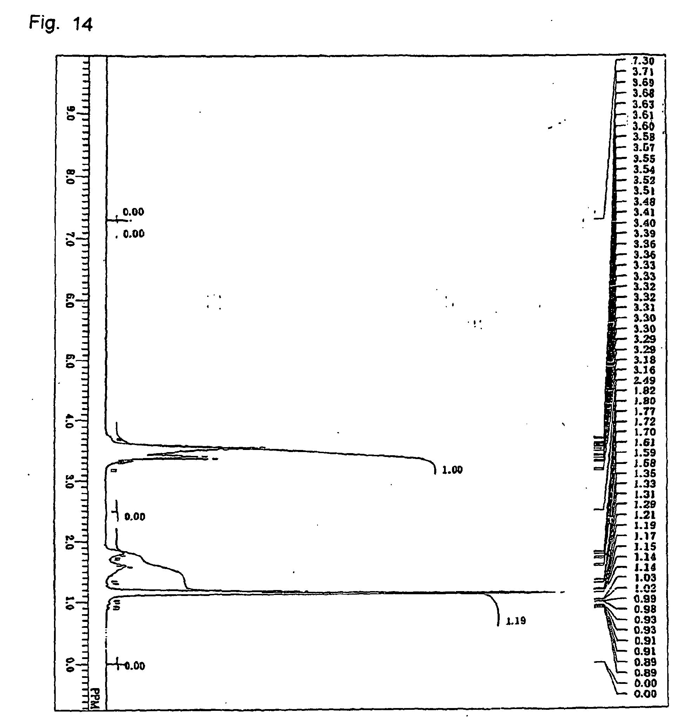

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 14 was obtained by the same way as that of Example 1.
The yield thereof was 95.4 g. A theoretical structure of Compound 14 estimated from the feed is (A) R^{y} = CH(CH₃)CH₂, m = 15.2 (average value), R^{z} = CH₃, (B) R^{x} = CH₂CH₃, (A) / (B) molar ratio (k/p) = 1/8, k+p = 9 (average value), and a calculated molecular weight of 1,518, from the formula (XX).
A ¹H-NMR chart of Compound 14 is as shown in Fig. 14.

### Example 15

### (Production of Vinyl Ether Polymerization Product)

A 2-liter separable flask made of glass was fed with 211.7 g of isooctane, 480.4 g (3.84×10⁻¹ mol) of polypropylene glycol monomethyl ether (having an average molecular weight of about 1,250), and 0.476 g of a boron trifluoride diethyl ether complex. Subsequently, 145.2 g (2.01 mol) of ethyl vinyl ether was added over 2 hours and 17 minutes. A reaction was exothermic, so a reaction solution was kept at 25°C by placing the flask in an ice-water bath. After that, 611.7 g of a crude product was obtained by the same way as that of Example 1. The crude product had kinematic viscosities of 124.2 mm²/s at 40°C and 21.02 mm²/s at 100°C.

### (Hydrogenation of Vinyl Ether Polymerization Product)

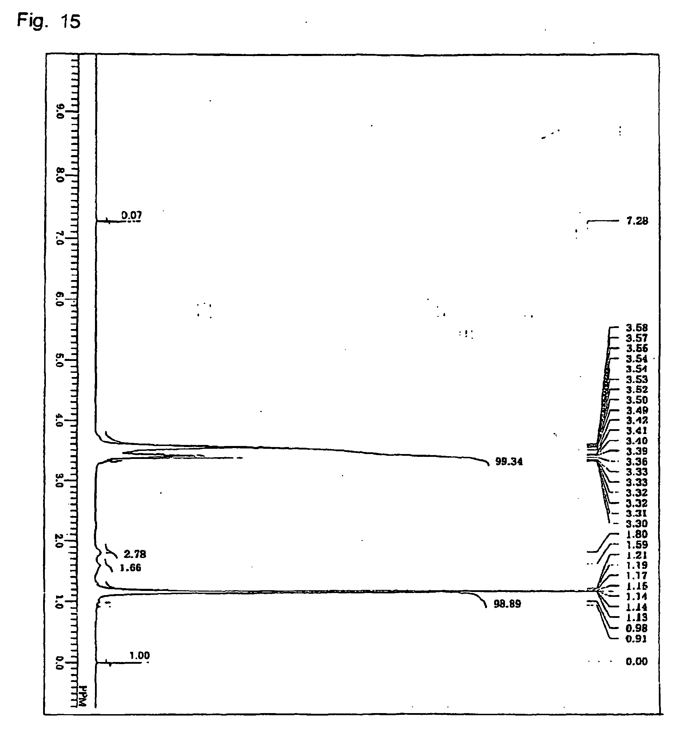

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 15 was obtained by the same way as that of Example 1. The yield thereof was 87.9g. A theoretical structure of Compound 15 estimated from the feed is (A) R^{y} = CH(CH₃)CH₂, m = 21.0 (average value), R^{z} = CH₃, (B) R^{x} = CH₂CH₃, (A) / (B) molar ratio (k/p) = 1/4, k+p = 5 (average value) , and a calculated molecular weight of 1, 566, from the formula (XX).
A ¹H-NMR chart of Compound 15 is as shown in Fig. 15.

### Example 16

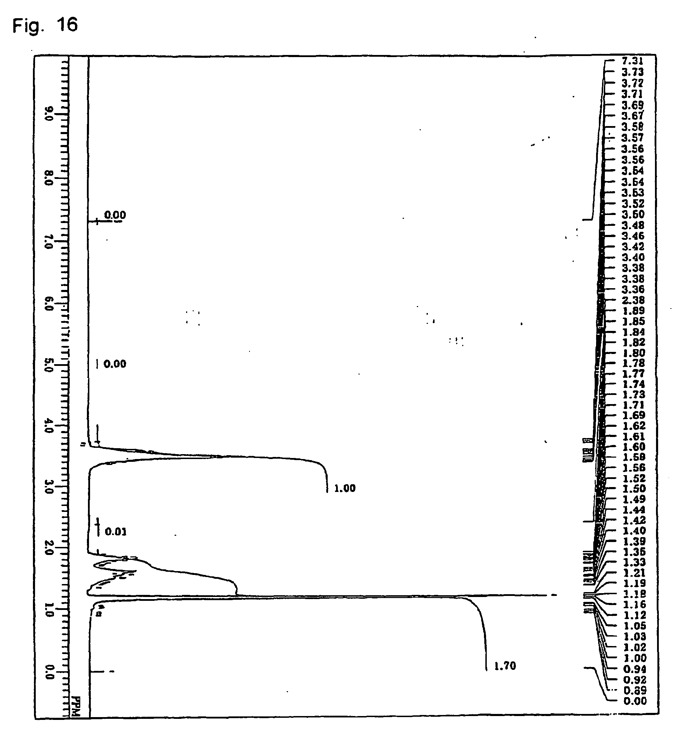

A 1-liter separable flask made of glass was fed with 60.5 g of toluene, 9.62 g (1.55×10⁻¹ mol) of ethylene glycol, and 0.366 g of a boron trifluoride diethyl ether complex. Subsequently, 190. 0 g (2.64 mol) of ethyl vinyl ether was added over 2 hours and 5 minutes. After that, 183.1 g of a crude product was obtained by the same way as that of Example 1. The crude product had kinematic viscosities of 81.79 mm²/s at 40°C and 8.629 mm²/s at 100°C.
Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 16 was obtained by the same way as that of Example 1. The yield thereof was 91. 9 g. A theoretical structure of Compound 16 estimated from the feed is R^{h} = CH₂CH₂, p = 1, R¹⁷ = CH₂CH₃, x+x' +y+y' = 15 (average value), and a calculated molecular weight of 1,200, from the formula (XXI) below.
A ¹H-NMR chart of Compound 16 is as shown in Fig. 16.

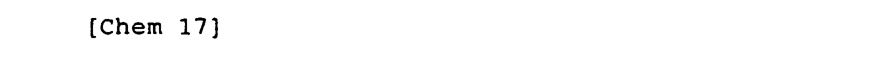

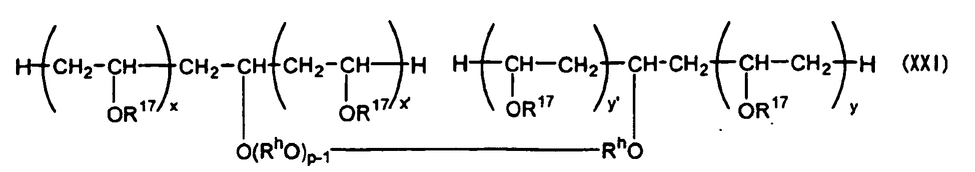

### Example 17

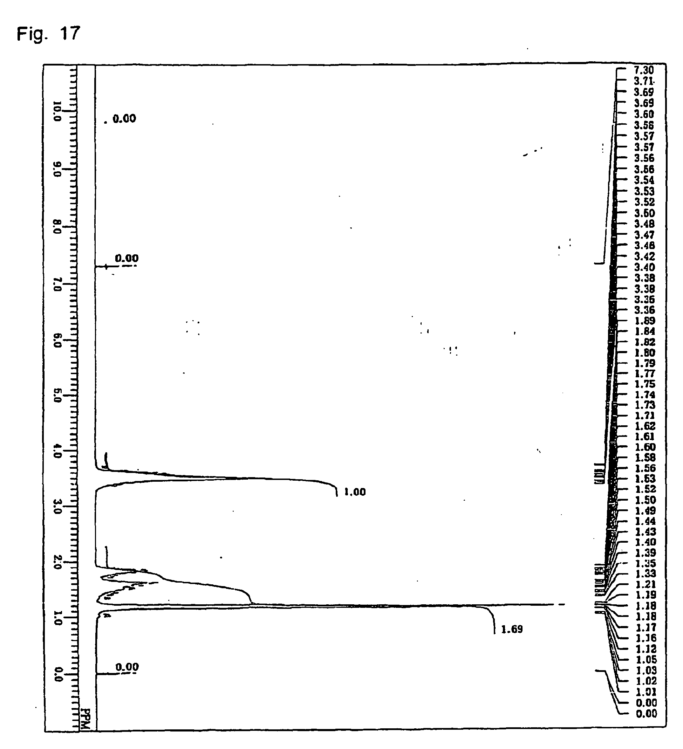

A 1-liter separable flask made of glass was fed with 60.5 g of toluene, 12.9 g (1.70x10⁻¹ mol) of propylene glycol, and 0.403 g of a boron trifluoride diethyl ether complex. Subsequently, 196.2 g (2.72 mol) of ethyl vinyl ether was added over 2 hours and 10 minutes. After that, 187.6 g of a crude product was obtained by the same way as that of Example 1. The crude product had kinematic viscosities of 84.62 mm²/s at 40°C and 8.713 mm²/s at 100°C.
Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 17 was obtained by the same way as that of Example 1. The yield thereof was 92.1 g. A theoretical structure of Compound 17 estimated from the feed is R^{h} = CH(CH₃)CH₂, p = 1, R¹⁷ = CH₂CH₃, x+x'+y+y' = 14 (average value), and a calculated molecular weight of 1,142, from the formula (XXI).
A ¹H-NMR chart of Compound 17 is as shown in Fig. 17.

### Example 18

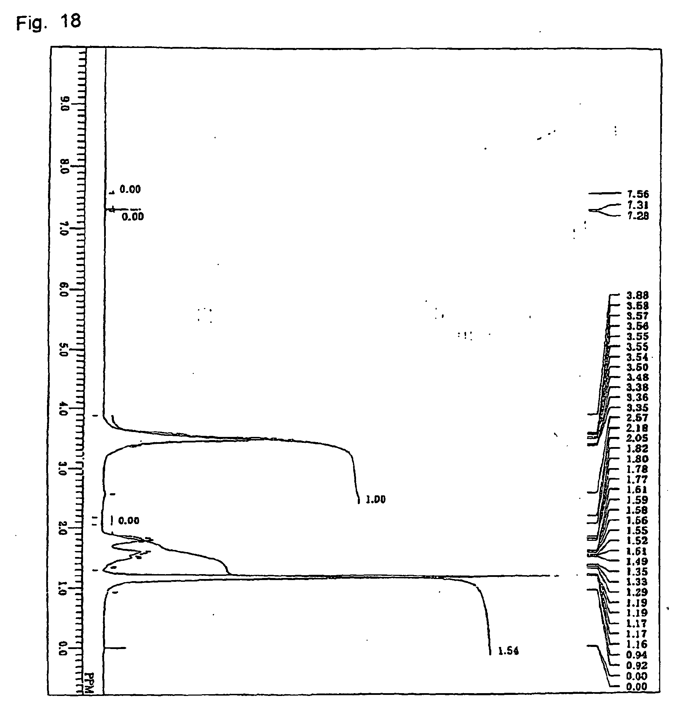

A 1-liter separable flask made of glass was fed with 60.5 g of toluene, 28.8 g (1.50×10⁻¹ mol) of tripropylene glycol, and 0.355 g of a boron trifluoride diethyl ether complex. Subsequently, 151. 4g (2.10 mol) of ethyl vinyl ether was added over 2 hours and 15 minutes. After that, 171.6 g of a crude product was obtained by the same way as that of Example 1. The crude product had kinematic viscosities of 132.3 mm²/s at 40°C and 13.00 mm²/s at 100°C.
Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 18 was obtained by the same way as that of Example 1. The yield thereof was 89. 2 g. A theoretical structure of Compound 18 estimated from the feed is R^{h} = CH(CH₃)CH₂, p = 3, R¹⁷ = CH₂CH₃, x+x'+y+y' = 12 (average value), and a calculated molecular weight of 1,114, from the formula (XXI).
A ¹H-NMR chart of Compound 18 is as shown in Fig. 18.

### Example 19

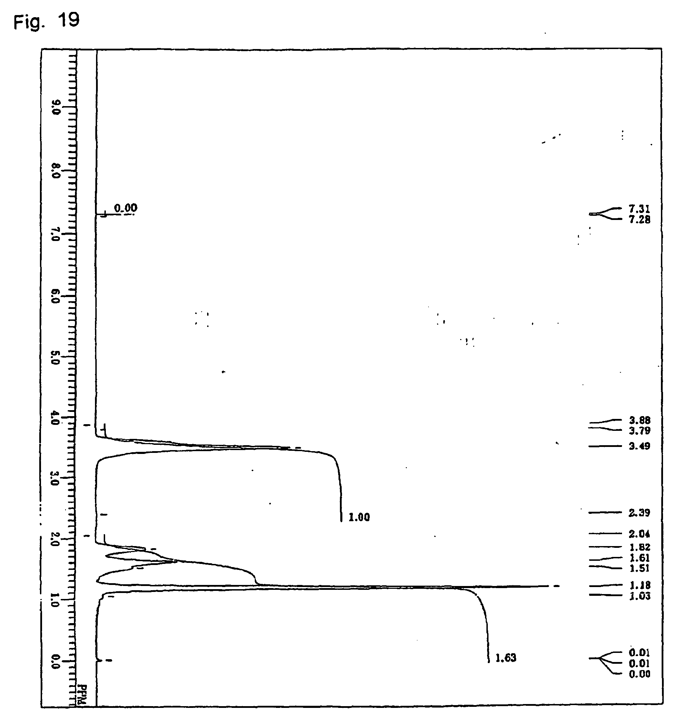

A 1-liter separable flask made of glass was fed with 60.6 g of toluene, 12.6 g (1.40×10⁻¹ mol) of 1,4-butandiol, and 0.332 g of a boron trifluoride diethyl ether complex. Subsequently, 171.6 g (2.38 mol) of ethyl vinyl ether was added over 2 hours and 32 minutes. After that, 177.1 g of a crude product was obtained by the same way as that of Example 1. The crude product had kinematic viscosities of 177.4 mm²/s at 40°C and 15.57 mm²/s at 100°C.
Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, and Compound 19 was obtained by the same way as that of Example 1. The yield thereof was 89. 8 g. A theoretical structure of Compound 19 estimated from the feed is R^{h} = CH₂CH₂CH₂CH₂, p = 1, R¹⁷ = CH₂CH₃, x+x' +y+y' = 15 (average value), and a calculated molecular weight of 1,228, from the formula (XXI).
A ¹H-NMR chart of Compound 19 is as shown in Fig. 19.

### Example 20

### (Production of Vinyl Ether Polymerization Product)

A 1-liter separable flask made of glass was fed with 52.5 g of isooctane, 23.1 g (5.00x10⁻¹ mol) of ethanol, and 0.592 g of a boron trifluoride diethyl ether complex. Subsequently, 216.5 g (3.00 mol) of ethyl vinyl ether was added over 3 hours. A reaction was exothermic, so a reaction solution was kept at 25°C by placing the flask in an ice-water bath. After the addition of all monomers, the reaction solution was continuously stirred for additional 20 minutes and 32.8 g (5.28×10⁻¹ mol) of ethylene glycol was then added and stirred for 5 minutes. The solvent and ethanol formed by the reaction were distilled off using a rotary evaporator. After that, the reaction solution was added with 50 g of isooctane and then transferred to a 2-liter washing tank, in which it was washed with 200 ml of a 3% by mass aqueous solution of sodium hydroxide and then washed with 200 ml of distilled water six times, followed by removing the solvent and light components using a rotary evaporator under reduced pressure. Consequently, 211.1 g of a crude product was obtained.

### (Hydrogenation of Vinyl Ether Polymerization Product)

Next, the autoclave containing the catalyst prepared in Catalyst Preparation Example 1 was opened and a liquid layer was then removed by decantation, followed by charging 300 g of isooctane and 91.0 g of the above-mentioned crude product. The autoclave was purged with nitrogen and then purged with hydrogen, followed by increasing the temperature therein while the pressure of hydrogen was adjusted to 3.0 MPaG. After retaining the autoclave at 160°C for 6 hours, the autoclave was cooled to room temperature. A decrease in hydrogen pressure was confirmed as the reaction proceeded while an increase in temperature allowed an increase in inner pressure of the autoclave. When the pressure of hydrogen decreases, hydrogen was suitably supplied, thereby keeping the inside of the autoclave at 3.0 MPaG. The autoclave was purged with nitrogen and then depressurized, followed by recovering a reaction solution and then removing the catalyst therefrom by filtration.
A filtrate was subjected to a rotary evaporator under reduced pressure to remove the solvent and light components. Consequently,83.3 g of a polyvinyl ether crude product having a hydroxyl group on an end was obtained. The crude product had kinematic viscosities of 40.46 mm²/s at 40°C and 5.661 mm²/s at 100°C.

### (Polymerization of Propylene Oxide)

A 30-ml eggplant type flask was fed with 0. 81 g of sodium hydride (oiliness, 60 to 72%) and an oil content was then removed by washing with hexane, followed by the addition of 45.00 g of the above-mentioned polyvinyl ether crude product having the hydroxyl group on the end. Upon the addition, evolution of bubbles was observed and sodium hydride was then dissolved. The solution was transferred to a 200-ml autoclave, 30 ml of triethylene glycol dimethyl ether and 23.2 g of propylene oxide were added thereto and the temperature thereof was then raised. It was kept at 110°C for 8 hours, followed by cooling down to room temperature. A decrease in hydrogen pressure was confirmed as the reaction proceeded while an increase in temperature allowed an increase in inner pressure of the autoclave.
A 300-ml eggplant type flask was fed with 5.25 g of sodium hydroxide (oiliness, 60 to 72%) and an oil content was then removed by washing with hexane, followed by the addition of 40 ml of triethylene glycol dimethyl ether and the above-mentioned polymerization solution. Upon the addition of the polymerization solution, evolution of bubbles was observed. Subsequently, 28.4 g (2.00×10⁻¹ mol) of methyl iodide was added over 2 hours and 30 minutes.
After completion of the addition of all of methyl iodide, the solution was continuously stirred for additional 5 hours. After that, a small amount of ethanol was added to confirm the absence of bubbling. Subsequently, the solution was added with 60 ml of isooctane and then transferred to a 500-ml separation funnel. After washing 10 times with 60 ml of pure water, the solvent was removed using the rotary evaporator under reduced pressure. Consequently, Compound 20 was obtained. The yield thereof was 64.1 g. A standard theoretical structure of Compound 20 estimated from the feed and the yield of the final product is represented by the following formula (XXII) and a calculated molecular weight of 644.

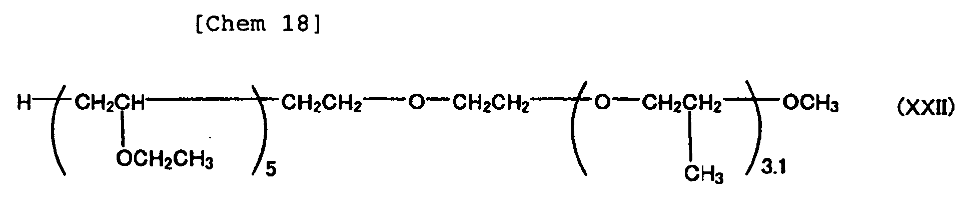

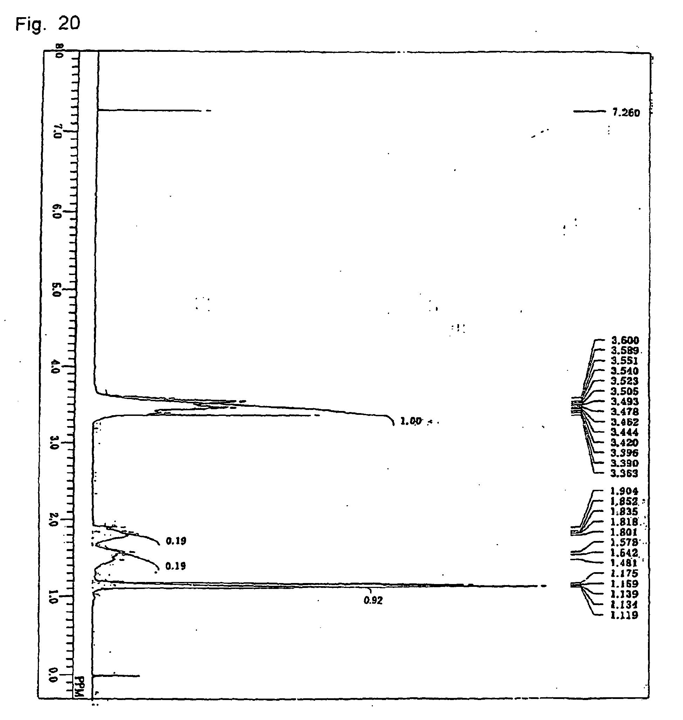

A ¹H-NMR chart of Compound 20 is as shown in Fig. 20.

### Comparative Example 1

### (Production of Vinyl Ether Polymerization Product)

A 2-liter separable flask made of glass was fed with 154 g of isooctane, 33.6 g (7.30×10⁻¹ mol) of ethanol, and 0.288 g of a boron trifluoride diethyl ether complex. Subsequently, 666.4 g (9.24 mol) of ethyl vinyl ether was added over 3 hours. After that, the reaction solution was transferred to a 2-liter washing tank, in which it was washed with 200 ml of a 3% by mass aqueous solution of sodium hydroxide and then washed with 200 ml of distilled water six times, followed by removing the solvent and light components using a rotary evaporator under reduced pressure. Consequently, 680.5 g of a crude product was obtained.
The crude product had kinematic viscosities of 123.5 mm²/s at 40°C and 11.08 mm²/s at 100°C.

### (Hydrogenation of Vinyl Ether Polymerization Product)

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, followed by increasing the temperature therein while the pressure of hydrogen was adjusted to 3.0 MPaG. After retaining the autoclave at 140°C for 2 hours, the autoclave was cooled to room temperature. A decrease in hydrogen pressure was confirmed as the reaction proceeded while an increase in temperature allowed an increase in inner pressure of the autoclave. When the pressure of hydrogen decreases, hydrogen was suitably supplied, thereby keeping the inside of the autoclave at 3.0 MPaG. The autoclave was purged with nitrogen and then depressurized, followed by recovering a reaction solution and then removing the catalyst therefrom by filtration.
The filtrate was subjected to a rotary evaporator under reduced pressure to remove the solvent and light components, thereby obtaining Compound 21. The yield thereof was 92. 0 g. A theoretical structure of Compound 21 estimated from the feed is R⁴ = CH₂CH₃, R¹, R², R³ = H, k = 0, p = 12.7 (average value), from the formula (I).

### Comparative Example 2

Commercially available polyalkylene glycol (PAG oil) (manufactured by Idemitsu Kosan Co., Ltd., trade name: Daphne Hermetic Oil PS) was used as Compound 22.

### Comparative Example 3

A mixture of 10% by mass of tripropylene glycol monomethyl ether used as an initiator in Example 7 and 90% by mass of the polyvinyl ether compound prepared in Comparative Example 1 was used as Compound 23.

### Comparative Example 4

### (Production of Vinyl Ether Polymerization Product)

A 1-liter separable flask made of glass was fed with 125 g of isooctane, 14.2 g (3.09×10⁻¹ mol) of ethanol, and 0.122 g of a boron trifluoride diethyl ether complex. Subsequently, 420.0 g (5.82 mol) of ethyl vinyl ether was added over 5 hours. A reaction was exothermic, so a reaction solution was kept at 45°C by placing the flask in an ice-water bath. After that, the reaction solution was transferred to a 2-liter washing tank, in which it was washed with 100 ml of a 3% by mass aqueous solution of sodium hydroxide and then washed with 200 ml of distilled water six times, followed by removing the solvent and light components using a rotary evaporator under reduced pressure. Consequently, 416.8 g of a crude product was obtained. The crude product had kinematic viscosities of 420. 6 mm²/s at 40°C and 24.04 mm²/s at 100°C.

### (Hydrogenation of Vinyl Ether Polymerization Product)

Next, the autoclave containing the catalyst prepared in Catalyst Preparation Example 1 was opened and a liquid layer was then removed by decantation, followed by charging 300 g of isooctane and 100 g of the above-mentioned crude product. The autoclave was purged with nitrogen and then purged with hydrogen, followed by obtaining Compound 24 by the same way as that of Comparative Example 1. The yield was 92.6 g. A theoretical structure of Compound 24 estimated from the feed is R^{x} = CH₂CH₃, k = 0, p = 18.8 (average value from the formula (xx).

### Comparative Example 5

Commercially available polyalkylene glycol (PAG oil) (manufactured by Idemitsu Kosan Co., Ltd., trade name: Daphne Hermetic Oil PZ100S) was used as Compound 25.

### Comparative Example 6

A mixture of 10% by mass of polypropylene glycol monomethyl ether (having an average molecular weight of about 440) used as an initiator in Example 12 and 90% by mass of the polyvinyl ether compound prepared in Comparative Example 4 was used as Compound 26.

For Compounds 1 to 20 obtained in Examples 1 to 20 and Compounds 21 to 26 obtained in Comparative Examples 1 to 6, kinematic viscosities (at 40°C and 100°C), viscosity indexes, pour points, and volume resistivity were measured. The results thereof are listed in Tables 1 and 2. In addition, each of the compounds was subjected to an NMR analysis. The characteristics of the respective compounds were determined and evaluated by the following methods:
(1) Kinematic viscosity
   The kinematic viscosities of sample oil were measured at 100°C and 40°C on the basis of JIS K2283, respectively.
(2) Viscosity index
   From the obtained kinematic viscosities, a viscosity index was determined on the basis of JIS K2283.
(3) Pour point
   A pour point was measured on the basis of JIS K2269.
(4) Volume resistivity
   The sample was dried at 100°C for 1 hour under reduced pressure (0.3 to 0.8 mmHg), and charged into a liquid cell for determining a volume resistivity in a thermostatic bath at 80°C. It was retained in the thermostatic bath at 80°C for 40 minutes, followed by a measurement using a ultra-high resistance meter R8340 manufactured by Advantest Corporation at applied voltage of 250 V.
(5) NMR (¹H-NMR)
¹H-NMR was performed using JNM-AL400 (trade name) (400 MHz) manufactured by JEOL Ltd. With heavy chloroform as a solvent and tetramethylsilane as an internal standard.
Assignments of peaks and hydrogen atoms in a ¹H-NMR chart and the corresponding structures in the general formula (I) are represented in parentheses on the ends of the respective lines.

Around 1.17 ppm: -OCH₂CH₃ (R⁴)

-OCH₂CH(CH₃)O- (R^{b})

1.4 to 1.9 ppm: -CH₂CH(OR)- (R¹, R²)

3.36 ppm: -OCH₃ (R^{a})

3.25 to 3.65 ppm: -CH₂CH(OR)- (R³)

-OCH₂CH₃ (R⁴)

-OCH₂CH(CH₃)O- (R^{b})

In addition, a peak in a range of 1.4 to 1.9 ppm represents the end structure of the general formula (XII) or (XIII), and a peak in a range of 3.25 to 3.65 ppm represents the end structure of the general formula (XIV) or (XV).

**Table 1**

| Table 1 | | | | | |
|---|---|---|---|---|---|
| | | Kinematic viscosities (mm²/s) | | Viscosity index | Volume resistivity (Ω·cm) |
| | | @40°C | @100°C | | |
| Example 1 | Compound 1 | 102.5 | 11.48 | 99 | 6.7×10¹² |
| Example 2 | Compound 2 | 52.59 | 7.981 | 120 | 1.9×10¹² |
| Example 3 | Compound 3 | 63.97 | 9.851 | 138 | 4.2×10¹² |
| Example 4 | Compound 4 | 107.5 | 11.89 | 99 | 3.7×10¹² |
| Example 5 | Compound 5 | 89.08 | 10.56 | 101 | 3.8×10¹² |
| Example 6 | Compound 6 | 45.02 | 7.067 | 116 | 1.1×10¹² |
| Example 7 | Compound 7 | 116.0 | 12.72 | 102 | 4.3×10¹² |
| Example 8 | Compound 8 | 56.36 | 8.768 | 132 | 2.0×10¹² |
| Example 9 | Compound 9 | 50.44 | 8.770 | 154 | 1.2×10¹² |
| Example 10 | Compound 10 | 91.88 | 11.50 | 114 | 2.8×10¹² |
| Example 11 | Compound 11 | 93.82 | 12.60 | 130 | 1.6×10¹² |
| Example 16 | Compound 16 | 69.72 | 8.428 | 88 | 5.4×10¹² |
| Example 17 | Compound 17 | 69.46 | 8.335 | 86 | 1.0×10¹³ |
| Example 18 | Compound 18 | 75.15 | 9.568 | 105 | 9.0×10¹² |
| Example 19 | Compound 19 | 117.1 | 12.99 | 104 | 2.9×10¹² |
| Example 20 | Compound 20 | 33.06 | 6.315 | 145 | 4.7×10¹² |
| Comparative Example 1 | Compound 21 | 96.43 | 10.30 | 86 | 5.2×10¹² |
| Comparative Example 2 | Compound 22 | 45.08 | 10.12 | 221 | 3.7×10¹⁰ |
| Comparative Example 3 | Compound 23 | 55.13 | 7.463 | 96 | 2.8×10¹¹ |

| | | | | | |
|---|---|---|---|---|---|
| Note: Compound 23 is a mixture. | | | | | |

Table 2

**Table 2**

| | | Kinematic viscosities(mm²/s) | | Viscosity index | Volume resistivity (Ω · cm) |
|---|---|---|---|---|---|
| | | @40°C | @100°C | | |
| Example 12 | Compound 12 | 231.5 | 22.75 | 120 | 4.5×10¹² |
| Example 13 | Compound 13 | 184.0 | 21.61 | 140 | 1.9×10¹² |
| Example 14 | Compound 14 | 130.0 | 19.43 | 170 | 9.1×10¹¹ |
| Example 15 | Compound 15 | 115.0 | 20.10 | 199 | 6.0×10¹¹ |
| Comparative Example 4 | Compound 24 | 335.4 | 22.57 | 82 | 4.8×10¹³ |
| Comparative Example 5 | Compound 25 | 104.9 | 20.10 | 217 | 8.3×10¹⁰ |
| Comparative Example 6 | Compound 26 | 151.7 | 14.83 | 97 | 1.2×10¹¹ |

| | | | | | |
|---|---|---|---|---|---|
| Note: Compound 26 is a mixture. | | | | | |

In Table 1, among the physical properties of the compounds (base oil) in the examples and the comparative examples, those having kinematic viscosities of approximately 10 mm²/s at 100 °C were described. The compounds of the respective examples in Table 1 have high viscosity indexes and volume resistivity enough to be used as motor-integrated refrigerator oil. However, it is found that high viscosity index and high volume resistivity are incompatible in the polyvinyl ether compound 21 of Comparative Example 1 or the polyalkylene glycol compound 22 of Comparative Example 2 alone. In addition, from Comparative Example 3, it is also found that high viscosity index and high volume resistivity are incompatible when the polyvinyl ether compound is only mixed with the polyalkylene glycol compound.
In Table 2, among the physical properties of the compounds (base oil) in the examples and the comparative Examples, those having kinematic viscosities of approximately 20 mm²/s at 100°C were described. The compounds of the respective examples in Table 2 have high viscosity indexes and volume resistivity enough to be used as motor-integrated refrigerator oil. However, it is found that high viscosity index and high volume resistivity are incompatible in the polyvinyl ether compound 24 of Comparative Example 4 or the polyalkylene glycol compound 25 of Comparative Example 5 alone. In addition, from Comparative Example 6, it is also found that high viscosity index and high volume resistivity are incompatible when the polyvinyl ether compound is only mixed with the polyalkylene glycol compound.

### Industrial Applicability

The polyvinyl ether compound of the present invention has a high viscosity index and a volume resistivity enough to be used as motor-integrated refrigerator oil, as well as excellent compatibility with a natural refrigerant and excellent lubrication property. Therefore, it can be used as lubricating oil for a compression refrigerator for a natural refrigerant. In addition, because of excellent compatibility to various HFC refrigerants, it can be used as lubricating oil for a compression refrigerator for any of various HFC refrigerants.
Further, the polyvinyl ether compound of the present invention can be used in a mixture refrigerant provided as a mixture thereof with a natural refrigerant such as carbon dioxide, for example, any of mixture refrigerants composed of any one of a HFC refrigerant, a fluorine-containing ether refrigerant, and a fluorine-free ether refrigerant such as dimethyl ether and any one of natural refrigerants such as carbon dioxide, ammonia, and hydrocarbon.
Further, for improving compatibility to a refrigerant, it can be used as a mixture with any of other lubricating oil for compression refrigerators.

## Claims

1. A polyvinyl ether compound having a molecular weight in a range of 300 to 3,000 and comprising a structure represented by the general formula (I): 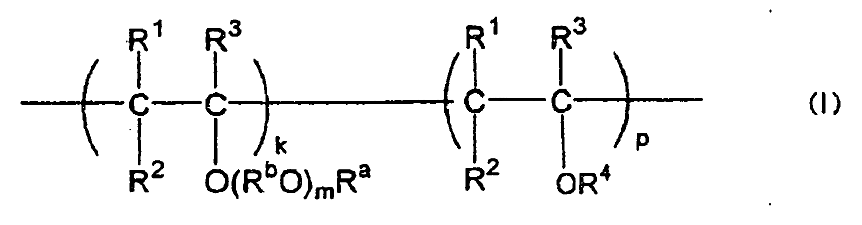 wherein R¹, R², and R³ each represent a hydrogen atom or a hydrocarbon group having 1 to 8 carbon atoms, which may be identical to or different from one another; R^{b} represents a divalent hydrocarbon group having 2 to 4 carbon atoms; R^{a} represents a hydrogen atom, an aliphatic or alicyclic hydrocarbon group having 1 to 20 carbon atoms, an aromatic group which has 1 to 20 carbon atoms and may have a substituent, an acyl group having 2 to 20 carbon atoms, or an oxygen-containing hydrocarbon group having 2 to 50 carbon atoms; R⁴ represents a hydrocarbon group having 1 to 10 carbon atoms; when there are two or more of each of R^{a}, R^{b}, and R⁴, they may be identical to or different from one another; m represents an average value of 2 to 50; k represents an average value of 1 to 50; p represents an average value of 0 to 50; when k and p each represent 2 or more, constitutional units may be in block or in random; and when there are two or more of R^{b}O, they may be identical to or different from one another.

2. A polyvinyl ether compound according to Claim 1, wherein R^{a} is an alkyl group having 1 to 4 carbon atoms.

3. A polyvinyl ether compound having a molecular weight in a range of 300 to 3,000 and comprising a structure represented by the general formula (II):
R^{c}-[[(OR^{d})ₐ-(A)_{b}-(OR^{f})ₑ]_{c}-R^{e}]_{d} (II)
wherein R^{c} represents a hydrogen atom, an alkyl group having 1 to 10 carbon atoms, an acyl group having 2 to 10 carbon atoms, or a hydrocarbon group having 1 to 10 carbon atoms and having 2 to 6 binding sites; each of R^{d} and R^{f} represents an alkylene group having 2 to 4 carbon atoms; each of a and e represents an average value of 0 to 50; c represents an integer of 1 to 20; R^{e} represents a hydrogen atom, an alkyl group having 1 to 10 carbon atoms, an alkoxy group having 1 to 10 carbon atoms, or an acyl group having 2 to 10 carbon atoms; and when a and/or e is 2 or more, (OR^{d}) and/or (OR^{f}) and (A) may be in random or in block; (A) is represented by the general formula (III): 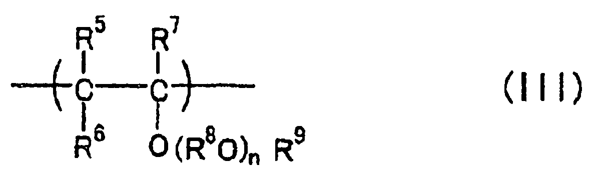 wherein R⁵, R⁶, and R⁷ each represent a hydrogen atom or a hydrocarbon group having 1 to 8 carbon atoms, which may be identical to or different from one another; R⁸ represents a divalent hydrocarbon group having 1 to 10 carbon atoms or a divalent hydrocarbon group containing ether-bonded oxygen and having 2 to 20 carbon atoms; R⁹ represents a hydrogen atom or a hydrocarbon group having 1 to 20 carbon atoms; n represents an average value of 0 to 10; when n represents 2 or more, constitutional units may be identical to or different from one another; R⁵ to R⁹ may be identical to or different from one another in every constitutional unit; and when there are two or more of R⁸O, they may be identical to or different from one another; b is 3 or more, d is an integer of 1 to 6; when a and e are zero (0), n represents an integer of 1 or more in one of the constitutional units (A).

4. A polyvinyl ether compound according to Claim 3, wherein R^{c} is a hydrogen atom, a = 0, and/or e = 0 in the general formula (II).

5. A polyvinyl ether compound according to Claim 4, wherein R^{e} is a hydrogen atom and c = 1 in the general formula (II).

6. A polyvinyl ether compound according to Claim 3, wherein, in the general formula (II), all of R⁵ to R⁷ are hydrogen atoms in (A), n is an average value of 0 to 4 and one of them is 1 or more, and R⁸ is a divalent hydrocarbon group having 2 to 4 carbon atoms.

7. A polyvinyl ether compound having a molecular weight in a range of 300 to 3,000 and comprising a structure represented by the general formula (IV):
R^{c}-[(OR^{d})ₐ- (A)_{b}- (OR^{f})ₑ]_{d}-R⁹ (IV)
wherein R^{c} represents a hydrogen atom, an alkyl group having 1 to 10 carbon atoms, an acyl group having 2 to 10 carbon atoms, or a hydrocarbon group having 1 to 10 carbon atoms and having 2 to 6 binding sites; each of R^{d} and R^{f} represents an alkylene group having 2 to 4 carbon atoms; each of a and e represents an average value of 0 to 50; (A) is represented by the general formula (III): 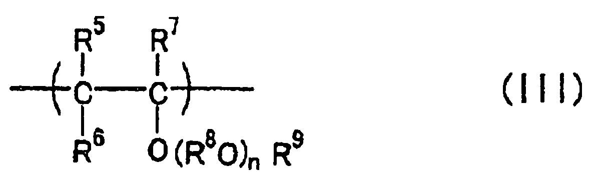 wherein R⁵, R⁶, and R⁷ each represent a hydrogen atom or a hydrocarbon group having 1 to 8 carbon atoms, which may be identical to or different from one another; R⁸ represents a divalent hydrocarbon group having 1 to 10 carbon atoms or a divalent hydrocarbon group containing ether-bonded oxygen and having 2 to 20 carbon atoms; R⁹ represents a hydrogen atom or a hydrocarbon group having 1 to 20 carbon atoms; n represents an average value of 0 to 10; when n represents 2 or more, constitutional units may be identical to or different from one another; R⁵ to R⁹ may be identical to or different from one another in every constitutional unit; and when there are two or more of R⁸O, they may be identical to or different from one another; b is 3 or more, d is an integer of 1 to 6; R⁹ represents a hydrogen atom, an alkyl group having 1 to 10 carbon atoms, an alkoxy group having 1 to 10 carbon atoms, an acyl group having 2 to 10 carbon atoms, or a hydrocarbon group having 1 to 10 carbon atoms and having 2 to 6 binding sites; when a and/or e is 2 or more, OR^{d} and/or OR^{f} and A may be in random or in block; and when both a and e are zero (0), n represents an integer of 1 or more in one of the structural units A.

8. A polyvinyl ether compound according to Claim 7, wherein R^{c} is a hydrogen atom and a = 0 in the general formula (IV).

9. A polyvinyl ether compound according to Claim 8, wherein R^{g} is a hydrogen atom, d = 1, and e = 0 in the general formula (IV).

10. A polyvinyl ether compound according to Claim 7, wherein, in the general formula (IV), all of R⁵ to R⁷ are hydrogen atoms in (A), n is an average value of 0 to 4 and one of them is 1 or more, and R⁸ is a divalent hydrocarbon group having 2 to 4 carbon atoms.

11. A polyvinyl ether compound having a molecular weight in a range of 300 to 3,000 and comprising a structure represented by the general formula (VI): 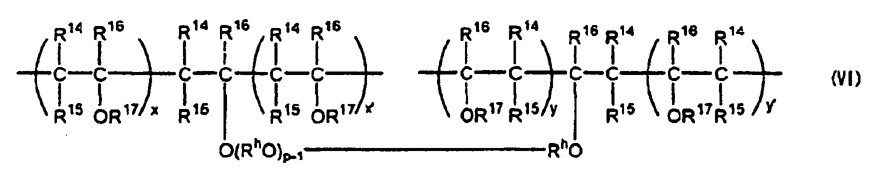 wherein R¹⁴, R¹⁵, and R¹⁶ each represent a hydrogen atom or a hydrocarbon group having 1 to 8 carbon atoms, which may be identical to or different from one another; R^{h} represents a divalent hydrocarbon group having 2 to 4 carbon atoms; R¹⁷ represents a hydrocarbon group having 1 to 10 carbon atoms; when there are two or more of each of R^{h} and R¹⁷ , they may be identical to or different from one another; p is an average value of 1 to 50; x+x' and y+y' each represent 1 to 50; and when each of x, x', y, and y' represents 2 or more, constitutional units may be in block or in random.

12. A polyvinyl ether compound according to Claim 1, comprising a polymerized product obtained by:
using as an initiator an alkylene glycol compound or a polyoxyalkylene compound represented by the general formula (VII): 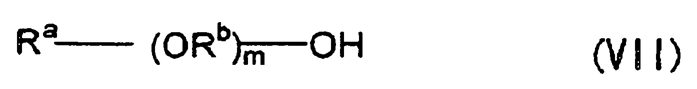
wherein R^{a} represents a hydrogen atom, an aliphatic or alicyclic hydrocarbon group having 1 to 20 carbon atoms, an aromatic group which has 1 to 20 carbon atoms and may have a substituent, an acyl group having 2 to 20 carbon atoms, or an oxygen-containing hydrocarbon group having 2 to 50 carbon atoms; R^{b} represents a divalent hydrocarbon group having 2 to 4 carbon atoms; and m represents an average value of 2 to 50; and
polymerizing vinyl ether compounds represented by the general formula (VIII): 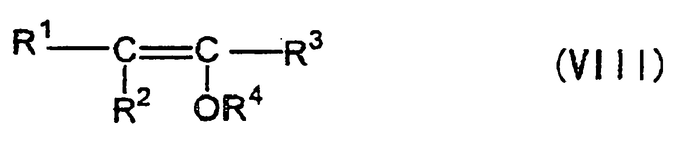
wherein R¹, R², and R³ each represent a hydrogen atom or a hydrocarbon group having 1 to 8 carbon atoms, which may be identical to or different from one another; and R⁴ represents a hydrocarbon group having 1 to 10 carbon atoms.

13. A polyvinyl ether compound according to Claim 1, comprising a polymerized product obtained by:
using as an initiator an acetal compound represented by the general formula (IX): 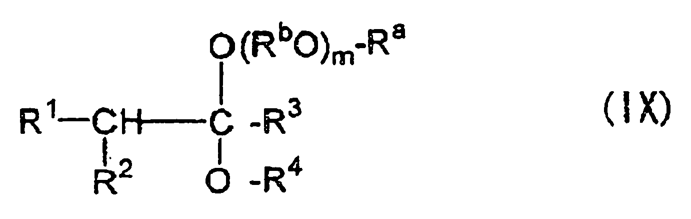
wherein, R¹ to R⁴, R^{a}, R^{b}, and m are as defined in Claim 1; R¹ to R³ may be identical to or different from one another; when there are two or more R^{b}O, the two or more R^{b}O may be identical to or different from one another; and
polymerizing the vinyl ether compounds represented by the general formula (VIII) as defined in Claim 12.

14. The polyvinyl ether compound according to Claim 1, comprising a polymerized product obtained by:
using as an initiator an acetal compound represented by the general formula (X): 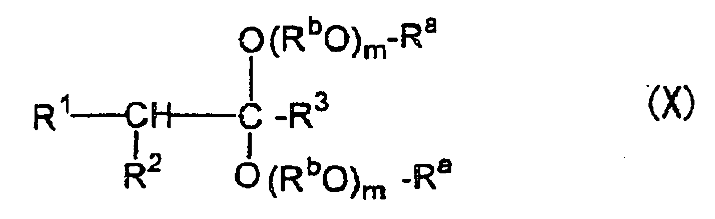
wherein, R¹ to R³, R^{a}, R^{b}, and m are as defined in Claim 1; R¹ to R³ may be identical to or different from one another; when there are two or more R^{b}O, the two or more R^{b}O may be identical to or different from one another; and
polymerizing the vinyl ether compounds represented by the general formula (VIII) as defined in Claim 12.

15. A polyvinyl ether compound according to Claim 1, comprising a polymerized product obtained such that an acetal compound represented by the general formula (X) as defined in Claim 14 and obtained by reacting one molecule of an alkylene glycol or polyoxyalkylene glycol compound represented by the general formula (VII) as defined in Claim 12 with one molecule of a vinyl ether compound represented by the general formula (XI) : 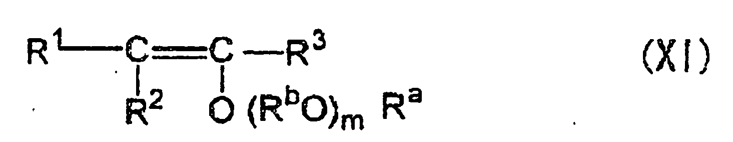 wherein R¹ to R³, R^{a}, R^{b}, and m are as defined in Claim 1; R¹ to R³ may be identical to or different from one another; when there are two or more R^{b}O, the two or more R^{b}O may be identical to or different from one another is isolated and used or directly used as an initiator, and the vinyl ether compounds represented by the general formula (VIII) as defined in Claim 12 are then polymerized.

16. A polyvinyl ether compound according to Claim 1, comprising a structure with one end represented by the general formula (XII) or (XIII): 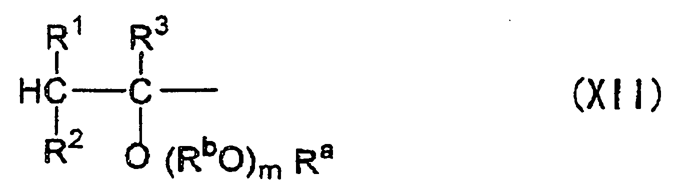 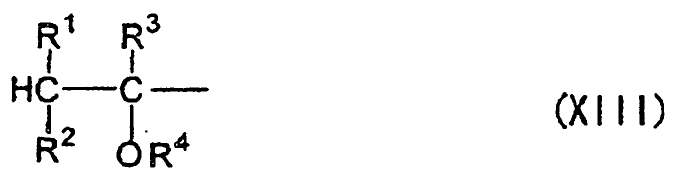 wherein R¹ to R⁴, R^{a}, R^{b}, and m are as defined in Claim 1; R¹ to R³ may be identical to or different from one another; when there are two or more R^{b}O, the two or more R^{b}O may be identical to or different from one another; and
another end represented by the general formula (XIV) or (XV) : 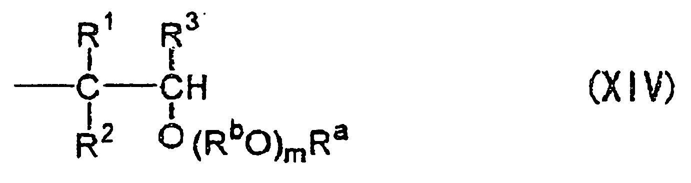 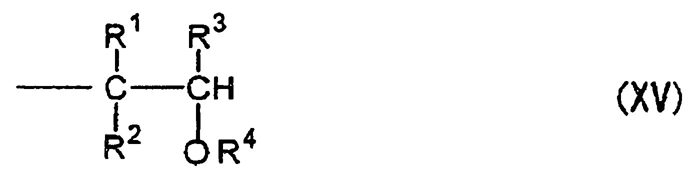 wherein R¹ to R³, R^{a}, R^{b}, and m are as defined in Claim 1; R¹ to R³ may be identical to or different from one another; when there are two or more R^{b}O, the two or more R^{b}O may be identical to or different from one another.

17. A polyvinyl ether compound according to Claim 11, comprising a polymerized product obtained by:
using as an initiator alkylene glycol or polyoxyalkylene glycol represented by the general formula (XVI): 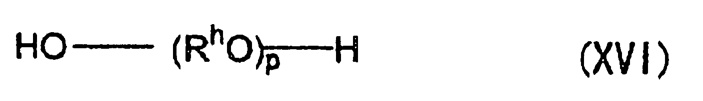
wherein R^{h} represents a divalent hydrocarbon group having 2 to 4 carbon atoms; and p represents an average value of 1 to 50; and
polymerizing vinyl ether compounds represented by the general formula (XVII): 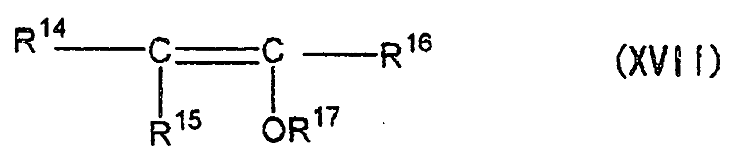
wherein R¹⁴, R¹⁵, and R¹⁶ each represent a hydrogen atom or a hydrocarbon group having 1 to 8 carbon atoms, which may be identical to or different from one another; and R¹⁷ represents a hydrocarbon group having 1 to 10 carbon atoms.

18. A process for preparing a polyvinyl ether compound according to any of Claims 1-11 comprising polymerizing vinyl ether compounds in the presence of a polymerization initiator, **characterized in that** at least one of the polymerization initiator and the vinyl ether compound contains an alkylene glycol residue or a polyoxyalkylene glycol residue.

## Patentansprüche

1. Polyvinylether-Verbindung, die ein Molekulargewicht im Bereich von 300 bis 3.000 besitzt und eine Struktur umfasst, die durch die allgemeine Formel (I) dargestellt wird: 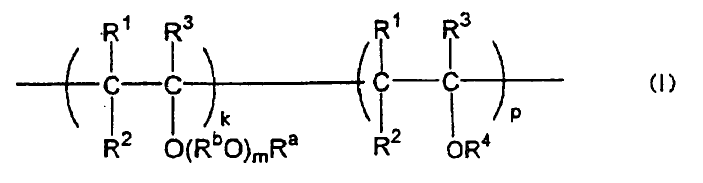 wobei R¹, R², und R³ jeweils ein Wasserstoffatom oder eine Kohlenwasserstoffgruppe mit 1 bis 8 Kohlenstoffatomen darstellen, die identisch oder verschieden voneinander sein können; R^{b} eine zweiwertige Kohlenwasserstoffgruppe mit 2 bis 4 Kohlenstoffatomen darstellt; R^{a} ein Wasserstoffatom, eine aliphatische oder alicyclische Kohlenwasserstoffgruppe mit 1 bis 20 Kohlenstoffatomen, eine aromatische Gruppe, die 1 bis 20 Kohlenstoffatome besitzt und einen Substituenten besitzen kann, eine Acylgruppe mit 2 bis 20 Kohlenstoffatomen oder eine sauerstoffhaltige Kohlenwasserstoffgruppe mit 2 bis 50 Kohlenstoffatomen darstellt; R⁴ eine Kohlenwasserstoffgruppe mit 1 bis 10 Kohlenwasserstoffatomen darstellt; wenn es zwei oder mehrere von jeweils R^{a}, R^{b} und R⁴ gibt, sie identisch oder voneinander verschieden sein können; m ein Mittelwert von 2 bis 50 darstellt; k ein Mittelwert von 1 bis 50 darstellt; p ein Mittelwert von 0 bis 50 darstellt; wenn k und p jeweils 2 oder mehr sind, konstitutionellen Einheiten blockweise oder statistisch vorliegen können; und wenn es 2 oder mehrere R^{b}O gibt, sie identisch oder voneinander verschieden sein können.

2. Polyvinylether-Verbindung nach Anspruch 1, wobei R^{a} eine Alkylgruppe mit 1 bis 4 Kohlenstoffatomen ist.

3. Polyvinylether-Verbindung, die ein Molekulargewicht im Bereich von 300 bis 3.000 besitzt und eine Struktur umfasst, die durch die allgemeine Formel (II) dargestellt wird:
**R^{c}-[[(OR^{d})ₐ-(A)_{b}-(OR^{f})ₑ]_{c}-R^{e}]_{d}** (II)
wobei R^{c} ein Wasserstoffatom, eine Alkylgruppe mit 1 bis 10 Kohlenstoffatomen, eine Acylgruppe mit 2 bis 10 Kohlenstoffatomen oder eine Kohlenwasserstoffgruppe mit 1 bis 10 Kohlenstoffatomen und mit 2 bis 6 Bindungsstellen darstellt; R^{d} und R^{f} jeweils eine Alkylengruppe mit 2 bis 4 Kohlenstoffatomen darstellen; a und e jeweils einen Mittelwert von 0 bis 50 darstellen; c eine ganze Zahl von 1 bis 20 darstellt; R^{e} ein Wasserstoffatom, eine Alkylgruppe mit 1 bis 10 Kohlenstoffatomen, eine Alkoxygruppe mit 1 bis 10 Kohlenstoffatomen oder eine Acylgruppe mit 2 bis 10 Kohlenstoffatomen darstellt; und wenn a und/oder e 2 oder mehr ist, (OR^{d}) und/oder (OR^{f}) und (A) statistisch oder blockweise vorliegen können; (A) wird durch die allgemeine Formel (III) dargestellt: 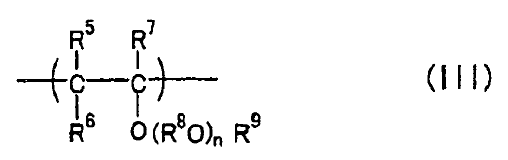 wobei R⁵, R⁶ und R⁷ jeweils ein Wasserstoffatom oder eine Kohlenwasserstoffgruppe mit 1 bis 8 Kohlenstoffatomen darstellen, die identisch oder voneinander verschieden sein können; R⁸ eine zweiwertige Kohlenwasserstoffgruppe mit 1 bis 10 Kohlenstoffatomen oder eine zweiwertige Kohlenwasserstoffgruppe, die einen Ethersauerstoff enthält und 2 bis 20 Kohlenstoffatome besitzt, darstellt; R⁹ ein Wasserstoffatom oder eine Kohlenwasserstoffgruppe mit 1 bis 20 Kohlenstoffatomen darstellt; n ein Mittelwert von 0 bis 10 darstellt; wenn n 2 oder mehr ist, konstitutionellen Einheiten identisch oder voneinander verschieden sein können; R⁵ bis R⁹ in jeder konstitutionellen Einheit identisch oder voneinander verschieden sein können; und wenn es zwei oder mehrere R⁸O gibt, sie identisch oder voneinander verschieden sein können; b 3 oder mehr ist, d eine ganze Zahl von 0 bis 6 ist; wenn a und e Null (0) sind, n eine ganze Zahl von 1 oder mehr in einer der konstitutionellen Einheiten (A) ist.

4. Polyvinylether-Verbindung nach Anspruch 3, wobei R^{c} ein Wasserstoffatom, a = 0 und/oder e = 0 in der allgemeinen Formel (II) ist.

5. Polyvinylether-Verbindung nach Anspruch 4, wobei, in der allgemeinen Formel (II), R^{e} ein Wasserstoffatom und c = 1 ist.

6. Polyvinylether-Verbindung nach Anspruch 3, wobei, in der allgemeinen Formel (II), alle R⁵ bis R⁷ Wasserstoffatome in (A) sind, n ein Mittelwert von 0 bis 4 ist und einer von ihnen 1 oder mehr ist, und R⁸ eine zweiwertige Kohlenwasserstoffgruppe mit 2 bis 4 Kohlenstoffatomen ist.

7. Polyvinyletherverbindung, die ein Molekulargewicht im Bereich von 300 bis 3.000 besitzt und eine Struktur umfasst, die durch die allgemeine Formel (IV) dargestellt wird:
**R^{c}-[(OR^{d})ₐ-(A)_{b}-(OR^{f})ₑ]_{d}-R^{g}** (IV)
wobei R^{c} ein Wasserstoffatom, eine Alkylgruppe mit 1 bis 10 Kohlenstoffatomen, eine Acylgruppe mit 2 bis 10 Kohlenstoffatomen oder eine Kohlenwasserstoffgruppe mit 1 bis 10 Kohlenstoffatomen und mit 2 bis 6 Bindungsstellen darstellt; R^{d} und R^{f} jeweils eine Alkylengruppe mit 2 bis 4 Kohlenstoffatomen darstellen; a und e jeweils einen Mittelwert von 0 bis 50 darstellen; (A) wird durch die allgemeine Formel (III) dargestellt: 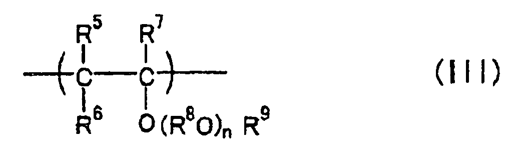 wobei R⁵, R⁶ und R⁷ jeweils ein Wasserstoffatom oder eine Kohlenwasserstoffgruppe mit 1 bis 8 Kohlenstoffatomen darstellen, die identisch oder voneinander verschieden sein können; R⁸ eine zweiwertige Kohlenwasserstoffgruppe mit 1 bis 10 Kohlenstoffatomen oder eine zweiwertige Kohlenwasserstoffgruppe, die einen Ethersauerstoff enthält und 2 bis 20 Kohlenstoffatome besitzt, darstellt; R⁹ ein Wasserstoffatom oder eine Kohlenwasserstoffgruppe mit 1 bis 20 Kohlenstoffatomen darstellt; n ein Mittelwert von 0 bis 10 darstellt, wenn n 2 oder mehr ist, konstitutionellen Einheiten identisch oder voneinander verschieden sein können; R⁵ bis R⁹ in jeder konstitutionellen Einheit identisch oder voneinander verschieden sein können; und wenn es zwei oder mehrere R⁸O gibt, sie identisch oder voneinander verschieden sein können; b 3 oder mehr ist, d eine ganze Zahl von 1 bis 6 ist; R⁹ ein Wasserstoffatom, eine Alkylgruppe mit 1 bis 10 Kohlenstoffatomen, eine Alkoxygruppe mit 1 bis 10 Kohlenstoffatomen, eine Acylgruppe mit 2 bis 10 Kohlenstoffatomen, oder eine Kohlenwasserstoffgruppe mit 1 bis 10 Kohlenstoffatomen und 2 bis 6 Bindungsstellen darstellt; wenn a und/oder e 2 oder mehr ist, OR^{d} und/oder OR^{f} und A statistisch oder blockweise vorliegen können; und wenn sowohl a als auch e Null (0) sind, n eine ganze Zahl von 1 oder mehr in einer der strukturellen Einheiten A darstellt.

8. Polyvinylether-Verbindung nach Anspruch 7, wobei R^{c} in der allgemeinen Formel (IV) ein Wasserstoffatom und a = 0 ist.

9. Polyvinylether-Verbindung nach Anspruch 8, wobei R⁹ in der allgemeinen Formel (IV) ein Wasserstoffatom, d = 1, und e = 0 ist.

10. Polyvinylether-Verbindung nach Anspruch 7, wobei, in der allgemeinen Formel (IV), alle R⁵ bis R⁷ Wasserstoffatome in (A), n ein Mittelwert von 0 bis 4 und einer von ihnen 1 oder mehr ist, und R⁸ eine zweiwertige Kohlenwasserstoffgruppe mit 2 bis 4 Kohlenstoffatomen ist.

11. Polyvinylether-Verbindung, die ein Molekulargewicht im Bereich von 300 bis 3.000 besitzt und eine Struktur umfasst, die durch die allgemeine Formel (VI) dargestellt wird: 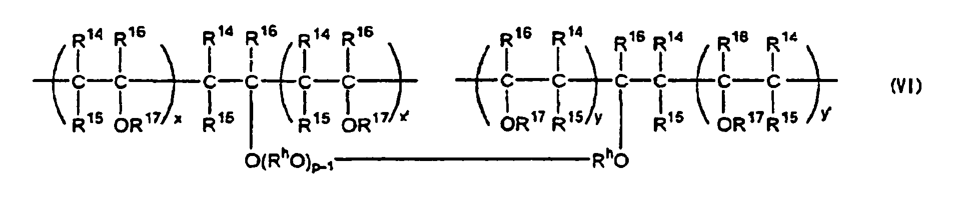 wobei R¹⁴, R¹⁵ und R¹⁶ jeweils ein Wasserstoffatom oder eine Kohlenwasserstoffgruppe mit 1 bis 8 Kohlenstoffatomen darstellen, die identisch oder voneinander verschieden sein können; R^{h} eine zweiwertige Kohlenwasserstoffgruppe mit 2 bis 4 Kohlenstoffatomen darstellt; R¹⁷ eine Kohlenwasserstoffgruppe mit 1 bis 10 Kohlenstoffatomen darstellt; wenn es zwei oder mehrere von je R^{h} und R¹⁷ gibt, sie identisch oder verschieden voneinander sein können; p ein Mittelwert von 1 bis 50 ist; x + x' und y + y' jeweils 1 bis 50 sind; und wenn jeweils x, x', y und y' 2 oder mehr sind, konstitutionellen Einheiten blockweise oder statistisch vorliegen können.

12. Polyvinylether-Verbindung nach Anspruch 1, umfassend ein Polymerisationsprodukt, erhalten durch:
Verwendung einer Alkylenglykol-Verbindung oder einer Polyoxyalkylen-Verbindung als ein Initiator, dargestellt durch die allgemeine Formel (VII): 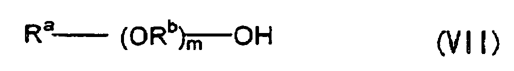
wobei R^{a} ein Wasserstoffatom, eine aliphatische oder alicyclische Kohlenwasserstoffgruppe mit 1 bis 20 Kohlenstoffatomen, eine aromatische Gruppe, die 1 bis 20 Kohlenstoffatome besitzt und einen Substituenten haben kann, eine Acylgruppe mit 2 bis 20 Kohlenstoffatomen oder eine sauerstoffhaltige Kohlenwasserstoffgruppe mit 2 bis 50 Kohlenstoffatomen darstellt; R^{b} eine zweiwertige Kohlenwasserstoffgruppe mit 2 bis 4 Kohlenstoffatomen darstellt; und m ein Mittelwert von 2 bis 50 darstellt; und
Polymerisieren der Vinylether-Verbindungen, die durch die allgemeine Formel (VIII) dargestellt werden: 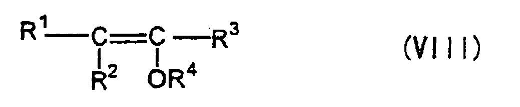
wobei R¹, R² und R³ jeweils ein Wasserstoffatom oder eine Kohlenwasserstoffgruppe mit 1 bis 8 Kohlenstoffatomen darstellen, die identisch oder voneinander verschieden sein können; und R⁴ eine Kohlenwasserstoffgruppe mit 1 bis 10 Kohlenstoffatomen darstellt.

13. Polyvinylether-Verbindung nach Anspruch 1, umfassend ein Polymerisationsprodukt, erhalten durch:
Verwendung einer Acetal-Verbindung als ein Initiator, die durch die allgemeine Formel (IX) dargestellt wird: 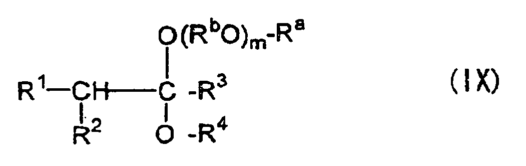
wobei R¹ bis R⁴, R^{a}, R^{b} und m wie in Anspruch 1 definiert sind; R¹ bis R³ identisch oder voneinander verschieden sein können; wenn es zwei oder mehrere R^{b}O gibt, die zwei oder mehreren R^{b}O identisch oder voneinander verschieden sein können; und
Polymerisieren der Vinylether-Verbindungen, dargestellt durch die allgemeine Formel (VIII), wie in Anspruch 12 definiert.

14. Polyvinylether-Verbindung nach Anspruch 1, umfassend ein Polymerisationsprodukt, erhalten durch:
Verwendung einer Acetal-Verbindung als ein Initiator, dargestellt durch die allgemeine Formel (X): 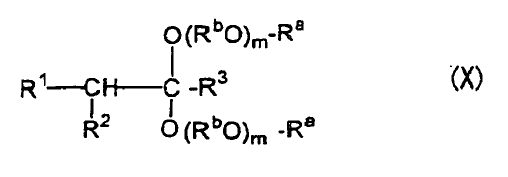
wobei R¹ bis R³, R^{a}, R^{b} und m wie in Anspruch 1 definiert sind; R¹ bis R³ identisch oder voneinander verschieden sein können; wenn es zwei oder mehrere R^{b}O gibt, die zwei oder mehreren R^{b}O identisch oder voneinander verschieden sein können; und
Polymerisieren der Vinylether-Verbindungen, dargestellt durch die allgemeine Formel (VIII), wie in Anspruch 12 definiert.

15. Polyvinylether-Verbindung nach Anspruch 1, umfassend ein Polymerisationsprodukt, welches so erhalten wird, dass eine Acetal-Verbindung, dargestellt durch die allgemeine Formel (X), wie in Anspruch 14 definiert, und erhalten durch Umsetzen eines Moleküls einer Alkylenglykol- oder Polyoxyalkylenglykol-Verbindung, dargestellt durch die allgemeine Formel (VII), wie in Anspruch 12 definiert, mit einem Molekül einer Vinylether-Verbindung, dargestellt durch die allgemeine Formel (XI): 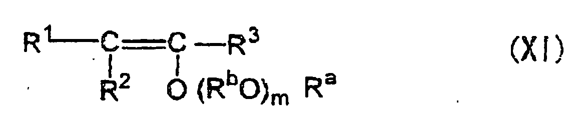 wobei R¹ bis R³, R^{a}, R^{b} und m wie in Anspruch 1 definiert sind; R¹ bis R³ identisch oder voneinander verschieden sein können; wenn es zwei oder mehrere R^{b}O gibt, die zwei oder mehreren R^{b}O identisch oder voneinander verschieden sein können, isoliert wird und als ein Initiator eingesetzt wird oder direkt als Initiator eingesetzt wird, und die Vinylether-Verbindungen, dargestellt durch die allgemeine Formel (VIII), wie in Anspruch 12 definiert, anschließend polymerisiert werden.

16. Polyvinylether-Verbindung nach Anspruch 1, welche eine Struktur mit einem Ende umfasst, die durch die allgemeinen Formel (XII) oder (XIII) dargestellt werden: 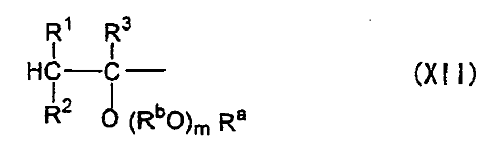 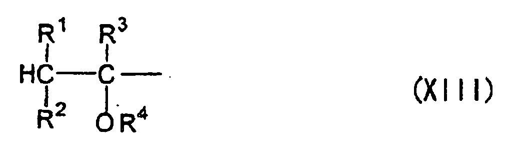 wobei R¹ bis R⁴, R^{a}, R^{b} und m wie in Anspruch 1 definiert sind; R¹ bis R³ identisch oder voneinander verschieden sein können; wenn es zwei oder mehrere R^{b}O gibt, die zwei oder mehreren R^{b}O identisch oder voneinander verschieden sein können; und
das andere Ende durch die allgemeine Formel (XIV) oder (XV) dargestellt wird: 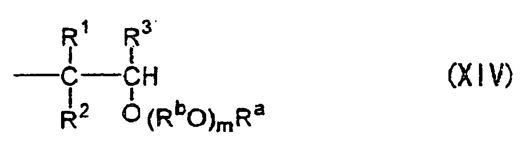 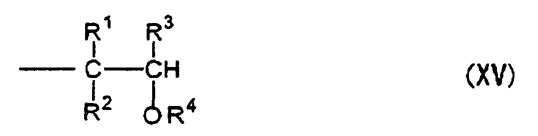 wobei R¹ bis R⁴, R^{a}, R^{b} und m wie in Anspruch 1 definiert sind; R¹ bis R³ identisch oder voneinander verschieden sein können; wenn es zwei oder mehrere R^{b}O gibt, die zwei oder mehreren R^{b}O identisch oder voneinander verschieden sein können.

17. Polyvinylether-Verbindung nach Anspruch 11, umfassend ein Polymerisationsprodukt, erhalten durch:
Verwendung eines Alkylenglykols oder Polyoxyalkylenglykols als Initiator dargestellt durch die allgemeine Formel (XVI): 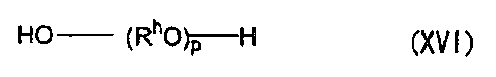
wobei R^{h} eine zweiwertige Kohlenwasserstoffgruppe mit 2 bis 4 Kohlenstoffatomen darstellt; und p ein Mittelwert von 1 bis 50 darstellt; und
Polymerisieren von Vinylether-Verbindungen, dargestellt durch die allgemeine Formel (XVII): 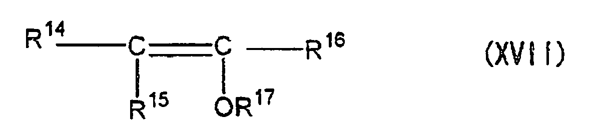
wobei R¹⁴, R¹⁵ und R¹⁶ jeweils ein Wasserstoffatom oder eine Kohlenwasserstoffgruppe mit 1 bis 8 Kohlenstoffatomen darstellen, die identisch oder voneinander verschieden sein können; und R¹⁷ eine Kohlenwasserstoffgruppe mit 1 bis 10 Kohlenstoffatomen darstellt.

18. Verfahren zur Herstellung einer Polyvinylether-Verbindung nach einem der Ansprüche 1 bis 11, umfassend Polymerisieren von Vinylether-Verbindungen in der Gegenwart eines Polymerisationsinitiators, **dadurch gekennzeichnet, dass** entweder der Polymerisationsinititator oder die Vinylether-Verbindung einen Alkylenglykolrest oder einen Polyoxyalkylenglykolrest enthalten.

## Revendications

1. Composé d'éther de polyvinyle ayant un poids moléculaire dans une plage de 300 à 3000 et comprenant une structure représentée par la formule générale (I): 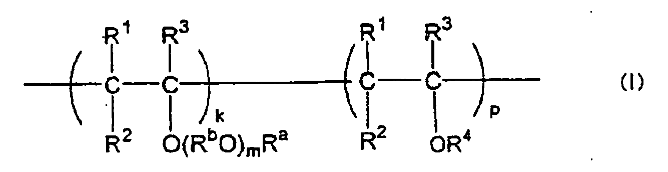 où R¹, R² et R³ représentent chacun un atome d'hydrogène ou un groupe hydrocarboné ayant 1 à 8 atomes de carbone, qui peuvent être identiques ou différents les uns des autres ; R^{b} représente un groupe hydrocarboné divalent ayant 2 à 4 atomes de carbone ; R^{a} représente un atome d'hydrogène, un groupe hydrocarboné aliphatique ou alicyclique ayant 1 à 20 atomes de carbone, un groupe aromatique qui a 1 à 20 atomes de carbone et peut avoir un substituant, un groupe acyle ayant 2 à 20 atomes de carbone, ou un groupe hydrocarboné contenant de l'oxygène ayant 2 à 50 atomes de carbone ; R⁴ représente un groupe hydrocarboné ayant 1 à 10 atomes de carbone ; lorsqu'il y a deux ou plus de chacun de R^{a}, R^{b}, et R⁴, ils peuvent être identiques ou différents les uns des autres ; m représente une valeur moyenne de 2 à 50 ; k représente une valeur moyenne de 1 à 50 ; p représente une valeur moyenne de 0 à 50 ; lorsque k et p représentent chacun 2 ou plus, les unités constituantes peuvent être en bloc ou dans un ordre aléatoire ; et lorsqu'il y a deux R^{b}O ou plus, ils peuvent être identiques ou différents les uns des autres.

2. Composé d'éther de polyvinyle selon la revendication 1, dans lequel R^{a} est un groupe alkyle ayant 1 à 4 atomes de carbone.

3. Composé d'éther de polyvinyle ayant un poids moléculaire dans une plage de 300 à 3000 et comprenant une structure représentée par la formule générale (II):
R^{c}-[[(OR^{d})ₐ-(A)_{b}-(OR^{f})ₑ]_{c}-R^{e}]_{d} (II)
où R^{c} représente un atome d'hydrogène, un groupe alkyle ayant 1 à 10 atomes de carbone, un groupe acyle ayant 2 à 10 atomes de carbone, ou un groupe hydrocarboné ayant 1 à 10 atomes de carbone et ayant 2 à 6 sites de liaison ; chacun de R^{d} et R^{f} représente un groupe alkylène ayant 2 à 4 atomes de carbone ; chacun de a et e représente une valeur moyenne de 0 à 50 ; c représente un nombre entier de 1 à 20 ; R^{e} représente un atome d'hydrogène, un groupe alkyle ayant 1 à 10 atomes de carbone, un groupe alcoxy ayant 1 à 10 atomes de carbone, ou un groupe acyle ayant 2 à 10 atomes de carbone ; et lorsque a et/ou e est 2 ou plus, (OR^{d}) et/ou (OR^{f}) et (A) peuvent être dans un ordre aléatoire ou en bloc ; (A) est représentée par la formule générale (III): 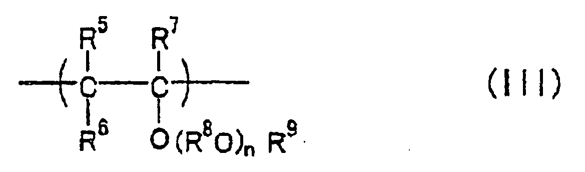 où R⁵, R⁶, et R⁷ représentent chacun un atome d'hydrogène ou un groupe hydrocarboné ayant 1 à 8 atomes de carbone, qui peuvent être identiques ou différents les uns des autres ; R⁸ représente un groupe hydrocarboné divalent ayant 1 à 10 atomes de carbone ou un groupe hydrocarboné divalent contenant un atome d'oxygène lié à l'éther et ayant 2 à 20 atomes de carbone ; R⁹ représente un atome d'hydrogène ou un groupe hydrocarboné ayant 1 à 20 atomes de carbone ; n représente une valeur moyenne de 0 à 10 ; lorsque n représente 2 ou plus, les unités constituantes peuvent être identiques ou différentes les unes des autres ; R⁵ à R⁹ peuvent être identiques ou différents les uns des autres dans chaque unité constituante ; et lorsqu'il y a deux R⁸O ou plus, ils peuvent être identiques ou différents les uns des autres ; b est 3 ou plus, d est un nombre entier de 1 à 6 ; lorsque a et e sont égaux à zéro (0), n représente un nombre entier de 1 ou plus dans l'une des unités constituantes (A).

4. Composé d'éther de polyvinyle selon la revendication 3, dans lequel R^{c} est un atome d'hydrogène, a=0, et/ou e=0 dans la formule générale (II).

5. Composé d'éther de polyvinyle selon la revendication 4, dans lequel R^{e} est un atome d'hydrogène et c=1 dans la formule générale (II).

6. Composé d'éther de polyvinyle selon la revendication 3, dans lequel, dans la formule générale (II), R⁵ à R⁷ sont des atomes d'hydrogène dans (A), n est une valeur moyenne de 0 à 4 et l'un d'eux est 1 ou plus, et R⁸ est un groupe hydrocarboné divalent ayant 2 à 4 atomes de carbone.

7. Composé d'éther de polyvinyle ayant un poids moléculaire dans une plage de 300 à 3000 et comprenant une structure représentée par la formule générale (IV):
R^{c}-[(OR^{d})ₐ-(A)_{b}-(OR^{f})ₑ]_{d}-R^{g} (IV)
où R^{c} représente un atome d'hydrogène, un groupe alkyle ayant 1 à 10 atomes de carbone, un groupe acyle ayant 2 à 10 atomes de carbone, ou un groupe hydrocarboné ayant 1 à 10 atomes de carbone et ayant 2 à 6 sites de liaison ; chacun de R^{d} et R^{f} représente un groupe alkylène ayant 2 à 4 atomes de carbone ; chacun de a et e représente une valeur moyenne de 0 à 50 ; (A) est représentée par la formule générale (III): 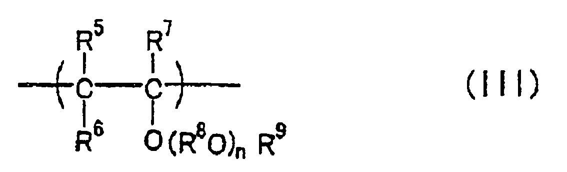 où R⁵, R⁶, et R⁷ représentent chacun un atome d'hydrogène ou un groupe hydrocarboné ayant 1 à 8 atomes de carbone, qui peuvent être identiques ou différents les uns des autres ; R⁸ représente un groupe hydrocarboné divalent ayant 1 à 10 atomes de carbone ou un groupe hydrocarboné divalent contenant un atome d'oxygène lié à l'éther et ayant 2 à 20 atomes de carbone ; R⁹ représente un atome d'hydrogène ou un groupe hydrocarboné ayant 1 à 20 atomes de carbone ; n représente une valeur moyenne de 0 à 10 ; lorsque n représente 2 ou plus, les unités constituantes peuvent être identiques ou différentes les unes des autres ; R⁵ à R⁹ peuvent être identiques ou différents les uns des autres dans chaque unité constituante ; et lorsqu'il y a deux R⁸O ou plus, ils peuvent être identiques ou différents les uns des autres, b est de 3 ou plus, d est un nombre entier de 1 à 6 ; R⁹ représente un atome d'hydrogène, un groupe alkyle ayant 1 à 10 atomes de carbone, un groupe alcoxy ayant de 1 à 10 atomes de carbone, un groupe acyle ayant 2 à 10 atomes de carbone, ou un groupe hydrocarboné ayant 1 à 10 atomes de carbone et ayant 2 à 6 sites de liaison ; lorsque a et/ou e est 2 ou plus, OR^{d} et/ou OR^{f} et A peuvent être dans un ordre aléatoire ou en bloc ; et lorsque à la fois a et e sont égaux à zéro (0), n représente un nombre entier de 1 ou plus dans l'une des unités structurelles A.

8. Composé d'éther de polyvinyle selon la revendication 7, dans lequel R^{c} est un atome d'hydrogène et a=0 dans la formule générale (IV).

9. Composé d'éther de polyvinyle selon la revendication 8, dans lequel R⁹ est un atome d'hydrogène, d=1, et e=0 dans la formule générale (IV).

10. Composé d'éther de polyvinyle selon la revendication 7, dans lequel, dans la formule générale (IV), R⁵ à R⁷ sont des atomes d'hydrogène dans (A), n est une valeur moyenne de 0 à 4 et l'un d'eux est 1 ou plus, et R⁸ est un groupe hydrocarboné divalent ayant 2 à 4 atomes de carbone.

11. Composé d'éther de polyvinyle ayant un poids moléculaire dans une plage de 300 à 3000 et comprenant une structure représentée par la formule générale (VI): 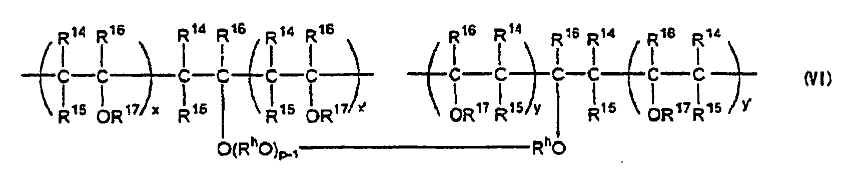 où R¹⁴, R¹⁵, et R¹⁶ représentent chacun un atome d'hydrogène ou un groupe hydrocarboné ayant 1 à 8 atomes de carbone, qui peuvent être identiques ou différents les uns des autres ; R^{h} représente un groupe hydrocarboné divalent ayant 2 à 4 atomes de carbone ; R¹⁷ représente un groupe hydrocarboné ayant 1 à 10 atomes de carbone ; lorsqu'il y a deux ou plus de chacun de R^{h} et R¹⁷, ils peuvent être identiques ou différents les uns des autres ; p est une valeur moyenne de 1 à 50 ; x+x' et y+y' représentent chacun 1 à 50 ; et lorsque chacun de x, x', y, et y' représente 2 ou plus, les unités constituantes peuvent être en bloc ou dans un ordre aléatoire.

12. Composé d'éther de polyvinyle selon la revendication 1, comprenant un produit polymérisé obtenu par :
l'utilisation, en tant qu'initiateur, d'un composé d'alkylène glycol ou d'un composé de polyoxyalkylène représenté par la formule générale (VII): 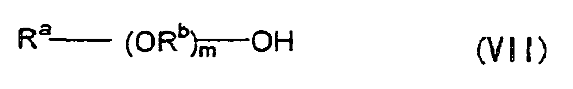
où R^{a} représente un atome d'hydrogène, un groupe hydrocarboné aliphatique ou alicyclique ayant 1 à 20 atomes de carbone, un groupe aromatique qui a 1 à 20 atomes de carbone et peut avoir un substituant, un groupe acyle ayant 2 à 20 atomes de carbone, ou un groupe hydrocarboné contenant un atome d'oxygène ayant 2 à 50 atomes de carbone ; R^{b} représente un groupe hydrocarboné divalent ayant 2 à 4 atomes de carbone ; et m représente une valeur moyenne de 2 à 50 ; et
la polymérisation des composés d'éthers de vinyle représentés par la formule générale (VIII): 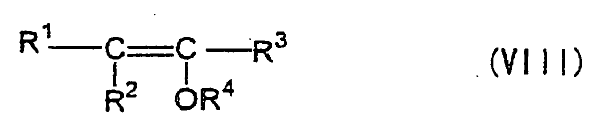
où R¹, R² et R³ représentent chacun un atome d'hydrogène ou un groupe hydrocarboné ayant 1 à 8 atomes de carbone, qui peuvent être identiques ou différents les uns des autres ; et R⁴ représente un groupe hydrocarboné ayant 1 à 10 atomes de carbone.

13. Composé d'éther de polyvinyle selon la revendication 1, comprenant un produit polymérisé obtenu par :
l'utilisation, en tant qu'initiateur, d'un composé d'acétal représenté par la formule générale (IX): 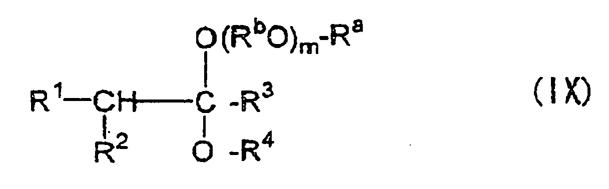
où, R¹ à R⁴, R^{a}, R^{b}, et m sont tels que définis dans la revendication 1 ; R¹ à R³ peuvent être identiques ou différents les uns des autres ; lorsqu'il y a deux R^{b}O ou plus, les deux R^{b}O ou plus peuvent être identiques ou différents les uns des autres ; et
la polymérisation des composés d'éthers de vinyle représentés par la formule générale (VIII) telle que définie dans la revendication 12.

14. Composé d'éther de polyvinyle selon la revendication 1, comprenant un produit polymérisé obtenu par :
l'utilisation, en tant qu'initiateur, d'un composé d'acétal représenté par la formule générale (X): 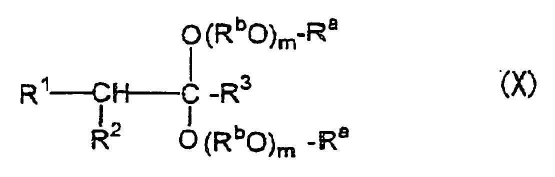
où, R¹ à R³, R^{a}, R^{b}, et m sont tels que définis dans la revendication 1, R¹ à R³ peuvent être identiques ou différents les uns des autres ; lorsqu'il y a deux R^{b}O ou plus, les deux R^{b}O ou plus peuvent être identiques ou différents les uns des autres ; et
la polymérisation des composés d'éthers de vinyle représentés par la formule générale (VIII) telle que définie dans la revendication 12.

15. Composé d'éther de polyvinyle selon la revendication 1 ; comprenant un produit polymérisé obtenu de sorte qu'un composé d'acétal représenté par la formule générale (X) telle que définie dans la revendication 14 et obtenu par la réaction d'une molécule d'un alkylène glycol ou d'un composé de polyoxyalkylène glycol représenté par la général formule (VII) telle que définie dans la revendication 12 avec une molécule d' un composé d'éther de vinyle représenté par la formule générale (XI): 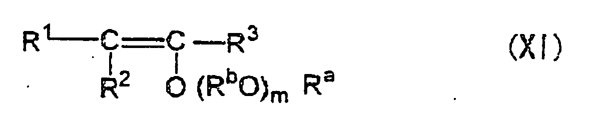 où R¹ à R³, R^{a}, R^{b}, et m sont tels que définis dans la revendication 1 ; R¹ à R³ peuvent être identiques ou différents les uns des autres ; lorsqu'il y a deux R^{b}O ou plus, les deux R^{b}O ou plus peuvent être identiques ou différents les uns des autres soit isolé et utilisé directement ou utilisé comme initiateur, et les composés d'éthers de vinyle représentés par la formule générale (VIII) telle que définie dans la revendication 12 sont ensuite polymérisés.

16. Composé d'éther de polyvinyle selon la revendication 1, comprenant une structure avec une extrémité représentée par la formule générale (XII) ou (XIII): 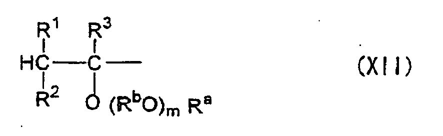 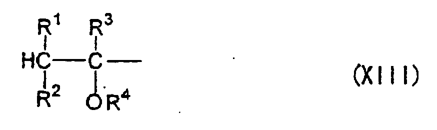 où R¹ à R⁴, R^{a}, R^{b}, et m sont tels que définis dans la revendication 1 ; R¹ à R³ peuvent être identiques ou différents les uns des autres ; lorsqu'il y a deux R^{b}O ou plus, les deux R^{b}O ou plus peuvent être identiques ou différents les uns des autres ; et
une autre extrémité représentée par la formule générale (XIV) ou (XV) : 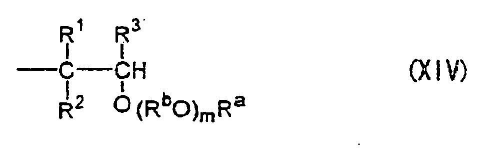 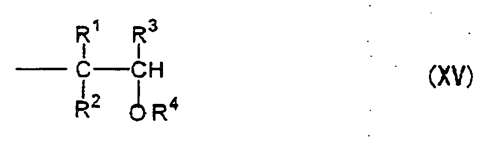 où R¹ à R³, R^{a}, R^{b}, et m sont tels que définis dans la revendication 1 ; R¹ à R³ peuvent être identiques ou différents les uns des autres ; lorsqu'il y a deux R^{b}O ou plus, les deux R^{b}O ou plus peuvent être identiques ou différents les uns des autres.

17. Composé d'éther de polyvinyle selon la revendication 11, comprenant un produit polymérisé obtenu par :
l'utilisation, en tant qu'initiateur, d'un alkylène glycol ou d'un polyoxyalkylène glycol représenté par la formule générale (XVI): 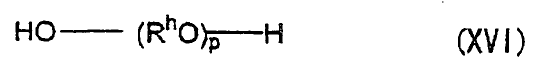
où R^{h} représente un groupe hydrocarboné divalent ayant 2 à 4 atomes de carbone ; et p représente une valeur moyenne de 1 à 50 ; et
la polymérisation des composés d'éthers de vinyle représentés par la formule générale (XVII): 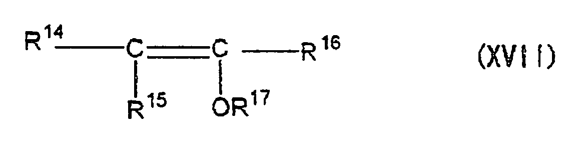
où R¹⁴, R¹⁵, et R¹⁶ représentent chacun un atome d'hydrogène ou un groupe hydrocarboné ayant 1 à 8 atomes de carbone, qui peuvent être identiques ou différents les uns des autres ; et R¹⁷ représente un groupe hydrocarboné ayant 1 à 10 atomes de carbone.

18. Processus de préparation d'un composé d'éther de polyvinyle selon l'une des revendications 1-11 comprenant la polymérisation des composés d'éther de vinyle en présence d'un initiateur de polymérisation, **caractérisé en ce qu'**au moins l'un de l'initiateur de polymérisation et du composé d'éther de vinyle contient un résidu d'alkylène glycol ou un résidu de polyoxyalkylène glycol.
